# EUROPEAN PATENT SPECIFICATION

(11) **EP 4 452 203 B1**
(45) Date of publication and mention of the grant of the patent: **24.12.2025**
(21) Application number: 22843305.8
(22) Date of filing: 21.12.2022
(51) Int. Cl.: A61K 8/34, A61Q 5/06, A61Q 5/10, A61K 8/41

(54) **PROCESS FOR DYEING KERATIN FIBRES USING A COSMETIC COMPOSITION COMPRISING PROPANE-1,3-DIOL AND A DYEING COMPOSITION**
VERFAHREN ZUR FÄRBUNG UNTER VERWENDUNG EINER KOSMETISCHEN ZUSAMMENSETZUNG MIT PROPAN-1,3-DIOL UND DEREN FÄRBEMITTELZUSAMMENSETZUNG
PROCÉDÉ DE COLORATION DES FIBRES KÉRATINIQUES METTANT EN UVRE UNE COMPOSITION COSMÉTIQUE COMPRENANT DU PROPANE-1,3-DIOL ET UNE COMPOSITION COLORANTE

(30) Priority: 22.12.2021 FR 2114263
(43) Date of publication of application: 30.10.2024
(73) Proprietor: L'OREAL, 75008 Paris (FR)
(72) Inventor: BRUYERE, Julie, 93400 SAINT OUEN (FR); WUNSCH, Karl, 93400 SAINT OUEN (FR); MARIO, Maud, 93400 SAINT OUEN (FR)
(74) Representative: Casalonga
(86) International application number: PCT/EP2022/087290
(87) International publication number: WO 2023/118333

(56) References cited:
- WO-A1-2008/061187
- WO-A1-2019/200027
- FR-A1- 2 925 317
- JP-A- H05 221 821

## Description

The present invention relates to a process for dyeing keratin fibres, in particular human keratin fibres such as the hair, comprising the application to said fibres of an extemporaneous mixture of at least one cosmetic composition comprising propane-1,3-diol and at least one dyeing composition.

The invention also relates to a multi-compartment device comprising at least one first compartment containing the cosmetic composition comprising propane-1,3-diol and at least one second compartment containing the dyeing composition.

In processes for dyeing keratin fibres, it is known to dye keratin fibres by different techniques starting from direct dyes for non-permanent colourings or oxidation dye precursors for permanent colourings.

Non-permanent dyeing or direct dyeing consists in dyeing keratin fibres with dyeing compositions containing direct dyes. These dyes are coloured and colouring molecules that have affinity for keratin fibres. They are applied to the keratin fibres for a time necessary to obtain the desired colouring, and are then rinsed out.

Some of these dyes may be used under lightening conditions, which enables the production of colourings that are visible on dark hair.

It is also known practice to dye keratin fibres permanently via oxidation dyeing. This dyeing technique consists in applying to the keratin fibres a composition containing dye precursors such as oxidation bases and couplers. Under the action of an oxidizing agent, these precursors form one or more coloured substances in the hair.

French Application FR-A-2925317 discloses Cosmetic composition, useful for oxidation colouring , lightening and bleaching of the keratin fibres , preferably hair, comprises alkanolamines, amino acids and polyoxyethylenated sorbitan esters and eventually propylene glycol (1,2-propane diol) as solvent.

International Application WO-A-2019/200027 discloses a composition and a process for modifying hair, the process includes coating hair fibres with a composition comprising propylene carbonate and a glycol selected from at least one of propylene glycol, 1,3-propane diol, dipropylene glycol, tripropylene glycol, and mixtures thereof and contacting the coated hair with a heating appliance at a temperature of at least 150°C for a sufficient time to modify the hair fibres .

However, the current dyeing processes can have a certain number of drawbacks. This is because, after application of the composition used in the process to keratin fibres, the dyeing power obtained may not be entirely satisfactory, indeed even be weak, and may result in a restricted range of colours. The colourings obtained may also be insufficiently persistent with respect to external agents, such as light, shampoos or perspiration, and may also be too selective, that is to say that the difference in colouring is too great along one and the same keratin fibre which is differently sensitized between its end and its root.

In addition, consumers are also in search of a dyeing process using compositions which are more environmentally friendly.

Thus, there exists a real need to make available a process for dyeing keratin fibres, in particular human keratin fibres such as the hair, which does not exhibit the abovementioned disadvantages, that is to say which is capable of resulting in good performance qualities, in particular in terms of colour build-up, of power and of chromaticity, while having a low selectivity and good persistence, for a wide range of shades.

One subject of the present invention is therefore a process for dyeing keratin fibres, in particular human keratin fibres such as the hair, comprising the application to said keratin fibres of an extemporaneous mixture:
i) of at least one cosmetic composition A comprising:
   - propane-1,3-diol, present in a total content of greater than or equal to 3% by weight, relative to the weight of the composition A,
   - one or more alkaline agents,
   - one or more dyes chosen from oxidation dyes, direct dyes and mixtures thereof, and
ii) of at least one dyeing composition B comprising:
   - one or more alkaline agents,
   - one or more dyes chosen from oxidation dyes, direct dyes and mixtures thereof;
it being understood that, when the compositions A and/or B comprise at least one oxidation dye, the extemporaneous mixture also comprises one or more oxidizing agents; said oxidizing agent(s) being present in the composition B and/or in an oxidizing composition C.

When said oxidizing agent(s) are present in the dyeing composition B, then said dyeing composition B preferably itself results from the mixing of a composition comprising one or more alkaline agents and one or more dyes chosen from oxidation dyes, direct dyes and mixtures thereof and of an additional oxidizing composition comprising one or more chemical oxidizing agents.

Preferably, when compositions A and/or B comprise at least one oxidation dye, the extemporaneous mixture comprises one or more oxidizing agents present in an oxidizing composition C.

Advantageously, the composition A is a booster composition, the composition B is a dyeing composition, optionally comprising an oxidizing agent, and the composition C is an oxidizing composition. The term "booster" composition is intended to mean that the composition A, comprising propane-1,3-diol, makes it possible in particular to improve the dyeing performance of the dyeing composition B.

The process according to the invention results in good dyeing properties with reduced contents of dye(s) and/or of oxidizing agent(s) during the dyeing process.

Propane-1,3-diol is in addition an environmentally friendly solvent which can be produced biologically. It thus also meets the demand of consumers for processes for dyeing keratin fibres, which are more environmentally friendly.

The process according to the invention thus makes it possible to achieve the above objectives, in particular in terms of colour build-up, of power of the colouring, of chromaticity, of selectivity and of persistence of the colouring, in particular with respect to shampoos, and also good working qualities, in particular easy application all along the fibre.

The process according to the invention also makes it possible to result in good cosmetic properties, in particular in terms of sheen and a more natural feel, and in a good level of comfort of the scalp.

The present invention also relates to a multi-compartment device comprising at least one first compartment containing the cosmetic composition A according to the invention as described above and at least one second compartment containing the composition B according to the invention.

Other subjects, characteristics, aspects and advantages of the invention will become even more clearly apparent on reading the description and the example which follows.

In the text hereinbelow, unless otherwise indicated, the limits of a range of values are included in that range, notably in the expressions "between" and "ranging from ... to ...".

Moreover, the expression "at least one" used in the present description is equivalent to the expression "one or more".

### Propane-1, 3-diol

The cosmetic composition A used in the process according to the invention comprises propane-1,3-diol.

The total content of propane-1,3-diol, present in the cosmetic composition A used in the process of the invention, is greater than or equal to 3% by weight, relative to the total weight of the cosmetic composition A. Preferably, the total content of propane-1,3-diol, present in the cosmetic composition A, ranges from 3% to 15% by weight, more preferentially from 4% to 10% by weight, even more preferentially from 4% to 7% by weight, relative to the total weight of the cosmetic composition A.

Preferably, composition B is different from composition A.

Preferably, composition B does not comprise propane-1,3-diol in a content greater than or equal to 3% by weight, relative to the total weight of the cosmetic composition B.

Preferably, composition B does not comprise propane-1,3-diol.

### Alkaline agent

The cosmetic composition A and the dyeing composition B used in the process according to the invention comprise one or more alkaline agent(s).

The alkaline agent(s) can be chosen from inorganic, organic or hybrid alkaline agents.

For the purposes of the present invention, the terms "alkaline agent" and "basifying agent" are used without distinction.

The inorganic basifying agent(s) are preferably chosen from aqueous ammonia, alkali metal or alkaline earth metal carbonates or bicarbonates, such as sodium (hydrogen)carbonate and potassium (hydrogen)carbonate, alkali metal or alkaline earth metal phosphates, such as sodium phosphates or potassium phosphates, sodium hydroxide or potassium hydroxide, alkali metal or alkaline earth metal silicates or metasilicates, such as sodium metasilicate, and mixtures thereof.

The organic basifying agent(s) are preferably chosen from alkanolamines, amino acids, organic amines other than alkanolamines, oxyethylenated and/or oxypropylenated ethylenediamines, 1,3-diaminopropane, spermine, spermidine and mixtures thereof.

The term "alkanolamine" means an organic amine comprising a primary, secondary or tertiary amine function, and one or more linear or branched C₁-C₈ alkyl groups bearing one or more hydroxyl radicals.

Organic amines chosen from alkanolamines such as monoalkanolamines, dialkanolamines or trialkanolamines comprising one to three identical or different C₁ to C₄ hydroxyalkyl radicals are in particular suitable for performing the invention.

In particular, the alkanolamine(s) are chosen from monoethanolamine (MEA), diethanolamine, triethanolamine, monoisopropanolamine, diisopropanolamine, N,N-dimethylethanolamine, 2-amino-2-methyl-1-propanol, triisopropanolamine, 2-amino-2-methyl-1,3-propanediol, 3-amino-1,2-propanediol, 3-dimethylamino-1,2-propanediol, tris(hydroxymethyl)aminomethane and mixtures thereof.

Advantageously, the amino acids are basic amino acids comprising an additional amine function. Such basic amino acids are preferably chosen from histidine, lysine, arginine, ornithine and citrulline.

The organic amine can also be chosen from organic amines of heterocyclic type. Mention may in particular be made, besides histidine already mentioned in the amino acids, of pyridine, piperidine, imidazole, triazole, tetrazole or benzimidazole. The organic amine can also be chosen from amino acid dipeptides. Mention may in particular be made, as amino acid dipeptides which can be used in the present invention, of carnosine, anserine and balenine. The organic amine can also be chosen from compounds comprising a guanidine function. Mention may in particular be made, as amines of this type other than arginine which can be used in the present invention, of creatine, creatinine, 1,1-dimethylguanidine, 1,1-diethylguanidine, glycocyamine, metformin, agmatine, N-amidinoalanine, 3-guanidinopropionic acid, 4-guanidinobutyric acid and 2-([amino(imino)methyl]amino)ethane-1-sulfonic acid.

Use may in particular be made, as hybrid compounds, of guanidine carbonate or monoethanolamine hydrochloride.

The alkaline agent(s) are preferably chosen from alkanolamines such as monoethanolamine, diethanolamine, triethanolamine; aqueous ammonia, carbonates or bicarbonates such as sodium (hydrogen)carbonate and potassium (hydrogen)carbonate, alkali metal or alkaline earth metal silicates or metasilicates such as sodium metasilicate and mixtures thereof, more preferentially from aqueous ammonia and alkanolamines, better still from alkanolamines, even better still the alkaline agent is monoethanolamine.

Preferably, the alkaline agent(s) are organic.

According to one particular embodiment, one of the compositions A and B according to the invention is free of aqueous ammonia.

According to another particular embodiment, the compositions A and B according to the invention are both free of aqueous ammonia.

The term "free of aqueous ammonia" is understood to mean that the cosmetic composition A and/or the dyeing composition B according to the invention do not comprise aqueous ammonia. In other words, the content of aqueous ammonia in the cosmetic composition A and/or the dyeing composition B is 0% by weight, relative to the weight of said composition.

Advantageously, the total content of the alkaline agent(s), present in the cosmetic composition A or in the dyeing composition B, ranges from 0.01% to 10% by weight, preferably from 0.05% to 8% by weight, more preferentially from 0.1% to 5% by weight, relative to the total weight of the composition comprising them.

Advantageously, the total content of the alkaline agent(s), present in the extemporaneous mixture of the compositions A and B of the process of the invention, ranges from 0.01% to 10% by weight, preferably from 0.05% to 8% by weight, more preferentially from 0.1% to 5% by weight, relative to the total weight of the mixture.

In a particular embodiment, the alkaline agent(s) are chosen from alkanolamines and mixtures thereof, preferably monoethanolamine, and the total content of the alkanolamine(s), present in the cosmetic composition A and/or the dyeing composition B according to the invention, preferably ranges from 0.01% to 10% by weight, more preferentially from 0.05% to 8% by weight, even more preferentially from 0.1% to 5% by weight, relative to the total weight of the composition comprising them.

According to this particular embodiment, the total content of the alkanolamine(s), present in the extemporaneous mixture applied in the process according to the invention, preferably ranges from 0.01% to 10% by weight, more preferentially from 0.05% to 8% by weight, even more preferentially from 0.1% to 5% by weight, relative to the total weight of the mixture.

According to one embodiment, the pH of the cosmetic composition A and of the dyeing composition B is between 5 and 8, preferably between 6 and 7.

The pH of the compositions A and B can be adjusted to the desired value by means of acidic or alkaline agent(s) commonly used in the dyeing of keratin fibres, such as those described above, or else using buffer systems known to those skilled in the art.

### Dyes

The cosmetic composition A and the dyeing composition B used in the dyeing process according to the invention also comprise one or more dyes chosen from oxidation dyes, direct dyes and mixtures thereof.

### The dyes: oxidation dyes

The oxidation dyes may be chosen from one or more oxidation bases, optionally in combination with one or more couplers.

Preferably, the cosmetic composition A and/or the dyeing composition B according to the invention comprise one or more oxidation bases.

The oxidation bases may be present in the form of salts, solvates and/or solvates of salts.

The addition salts of the oxidation bases present in the composition according to the invention are chosen notably from the addition salts with an acid, such as the hydrochlorides, hydrobromides, sulfates, citrates, succinates, tartrates, lactates, tosylates, benzenesulfonates, methanesulfonates, phosphates and acetates, and the addition salts with a base such as sodium hydroxide, potassium hydroxide, aqueous ammonia, amines or alkanolamines.

Moreover, the solvates of the additional oxidation bases more particularly represent the hydrates of said oxidation bases and/or the combination of said oxidation bases with a linear or branched C1 to C4 alcohol such as methanol, ethanol, isopropanol or n-propanol. Preferably, the solvates are hydrates.

The oxidation bases can be chosen from para-phenylenediamines, bisphenylalkylenediamines, para-aminophenols, ortho-aminophenols, heterocyclic bases and the addition salts thereof, the solvates thereof and solvates of the salts thereof, and mixtures thereof.

Mention may be made, among the para-phenylenediamines, by way of example, of para-phenylenediamine, para-tolylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-N,N-bis(β-hydroxyethyl)amino-2-methylaniline, 4-N,N-bis(β-hydroxyethyl)amino-2-chloroaniline, 2-β-hydroxyethyl-para-phenylenediamine, 2-methoxymethyl-para-phenylenediamine, 2-γ-hydroxypropyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N-ethyl-N-(β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-β-hydroxyethyloxy-para-phenylenediamine, 2-β-acetylaminoethyloxy-para-phenylenediamine, N-(β-methoxyethyl)-para-phenylenediamine, 4-aminophenylpyrrolidine, 2-thienyl-para-phenylenediamine, 2-β-hydroxyethylamino-5-aminotoluene, 3-hydroxy-1-(4'-aminophenyl)pyrrolidine and the addition salts thereof, the solvates thereof, and the solvates of the salts thereof.

Among the para-phenylenediamines mentioned above, para-phenylenediamine, para-tolylenediamine, 2-isopropyl-para-phenylenediamine, 2-β-hydroxyethyl-para-phenylenediamine, 2-methoxymethyl-para-phenylenediamine, 2-γ-hydroxypropyl-para-phenylenediamine, 2-β-hydroxyethyloxy-para-phenylenediamine, 2,6-dimethyl-para-phenylenediamine, 2,6-diethyl-para-phenylenediamine, 2,3-dimethyl-para-phenylenediamine, N,N-bis(β-hydroxyethyl)-para-phenylenediamine, 2-chloro-para-phenylenediamine and 2-β-acetylaminoethyloxy-para-phenylenediamine, and the addition salts thereof, the solvates thereof, and the solvates of the salts thereof are particularly preferred.

Among the bis(phenyl)alkylenediamines, examples that may be mentioned include N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-bis(ethyl)-N,N'-bis(4'-amino-3'-methylphenyl)ethylenediamine and 1,8-bis(2,5-diaminophenoxy)-3,6-dioxaoctane, and the addition salts thereof, the solvates thereof, and the solvates of the salts thereof.

Among the para-aminophenols, examples that may be mentioned include para-aminophenol, 4-amino-3-methylphenol, 4-amino-3-fluorophenol, 4-amino-3-chlorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol and 4-amino-2-fluorophenol, and the addition salts thereof, the solvates thereof, and the solvates of the salts thereof.

Among the ortho-aminophenols, examples that may be mentioned include 2-aminophenol, 2-amino-5-methylphenol, 2-amino-6-methylphenol and 5-acetamido-2-aminophenol, and the addition salts thereof, the solvates thereof, and the solvates of the salts thereof.

Among the heterocyclic bases, examples that may be mentioned include pyridine derivatives, pyrimidine derivatives and pyrazole derivatives.

Mention may be made, among the pyridine derivatives, of the compounds described, for example, in patents GB 1 026 978 and GB 1 153 196, such as 2,5-diaminopyridine, 2-(4-methoxyphenyl)amino-3-aminopyridine, 3,4-diaminopyridine and the addition salts thereof, the solvates thereof, and the solvates of the salts thereof.

Other pyridine oxidation bases that are useful in the present invention are the 3-aminopyrazolo[1,5-a]pyridine oxidation bases or the addition salts thereof described, for example, in patent application FR 2 801 308. Mention may be made, by way of example, of pyrazolo[1,5-a]pyridin-3-ylamine, 2-acetylaminopyrazolo[1,5-a]pyridin-3-ylamine, 2-(morpholin-4-yl)pyrazolo[1,5-a]pyridin-3-ylamine, 3-aminopyrazolo[1,5-a]pyridine-2-carboxylic acid, 2-methoxypyrazolo[1,5-a]pyridin-3-ylamine, (3-aminopyrazolo[1,5-a]pyridin-7-yl)methanol, 2-(3-aminopyrazolo[1,5-a]pyridin-5-yl)ethanol, 2-(3-aminopyrazolo[1,5-a]pyridin-7-yl)ethanol, (3-aminopyrazolo[1,5-a]pyridin-2-yl)methanol, 3,6-diaminopyrazolo[1,5-a]pyridine, 3,4-diaminopyrazolo[1,5-a]pyridine, pyrazolo[1,5-a]pyridine-3,7-diamine, 7-(morpholin-4-yl)pyrazolo[1,5-a]pyridin-3-ylamine, pyrazolo[1,5-a]pyridine-3,5-diamine, 5-(morpholin-4-yl)pyrazolo[1,5-a]pyridin-3-ylamine, 2-[(3-aminopyrazolo[1,5-a]pyridin-5-yl)(2-hydroxyethyl)amino]ethanol, 2-[(3-aminopyrazolo[1,5-a]pyridin-7-yl)(2-hydroxyethyl)amino]ethanol, 3-aminopyrazolo[1,5-a]pyridin-5-ol, 3-aminopyrazolo[1,5-a]pyridin-4-ol, 3-aminopyrazolo[1,5-a]pyridin-6-ol, 3-aminopyrazolo[1,5-a]pyridin-7-ol, 2-(3-aminopyrazolo[1,5-a]pyridin-2-yl)oxyethanol and also the addition salts thereof, the solvates thereof, and the solvates of the salts thereof.

Among the pyrimidine derivatives, mention may be made of the compounds described, for example, in patents DE 2359399, JP 88-169571, JP 05-63124 and EP 0770375 or patent application WO 96/15765, such as 2,4,5,6-tetraaminopyrimidine, 4-hydroxy-2,5,6-triaminopyrimidine, 2-hydroxy-4,5,6-triaminopyrimidine, 2,4-dihydroxy-5,6-diaminopyrimidine, 2,5,6-triaminopyrimidine and the addition salts thereof and the tautomeric forms thereof, when a tautomeric equilibrium exists.

Among the pyrazole derivatives that may be mentioned are the compounds described in patents DE 3843892 and DE 4133957 and patent applications WO 94/08969, WO 94/08970, FR-A-2 733 749 and DE 195 43 988, such as 4,5-diamino-1-methylpyrazole, 4,5-diamino-1-(β-hydroxyethyl)pyrazole, 3,4-diaminopyrazole, 4,5-diamino-1-(4'-chlorobenzyl)pyrazole, 4,5-diamino-1,3-dimethylpyrazole, 4,5-diamino-3-methyl-1-phenylpyrazole, 4,5-diamino-1-methyl-3-phenylpyrazole, 4-amino-1,3-dimethyl-5-hydrazinopyrazole, 1-benzyl-4,5-diamino-3-methylpyrazole, 4,5-diamino-3-tert-butyl-1-methylpyrazole, 4,5-diamino-1-tert-butyl-3-methylpyrazole, 4,5-diamino-1-(β-hydroxyethyl)-3-methylpyrazole, 4,5-diamino-1-ethyl-3-methylpyrazole, 4,5-diamino-1-ethyl-3-(4'-methoxyphenyl)pyrazole, 4,5-diamino-1-ethyl-3-hydroxymethylpyrazole, 4,5-diamino-3-hydroxymethyl-1-methylpyrazole, 4,5-diamino-3-hydroxymethyl-1-isopropylpyrazole, 4,5-diamino-3-methyl-1-isopropylpyrazole, 4-amino-5-(2'-aminoethyl)amino-1,3-dimethylpyrazole, 3,4,5-triaminopyrazole, 1-methyl-3,4,5-triaminopyrazole, 3,5-diamino-1-methyl-4-methylaminopyrazole and 3,5-diamino-4-(β-hydroxyethyl)amino-1-methylpyrazole, and the addition salts thereof, the solvates thereof, and the solvates of the salts thereof. Use may also be made of 4,5-diamino-1-(β-methoxyethyl)pyrazole.

Preferably, use will be made of a 4,5-diaminopyrazole and more preferentially still of 4,5-diamino-1-(β-hydroxyethyl)pyrazole and/or a salt thereof, a solvate thereof, a solvate of the salts thereof.

Pyrazole derivatives that may also be mentioned include diamino-N,N-dihydropyrazolopyrazolones and notably those described in patent application FR-A-2 886 136, such as the following compounds and the addition salts thereof: 2,3-diamino-6,7-dihydro-1H,5H-pyrazolo[1,2-a]pyrazol-1-one, 2-amino-3-ethylamino-6,7-dihydro-1H,5H-pyrazolo[1,2-a]pyrazol-1-one, 2-amino-3-isopropylamino-6,7-dihydro-1H,5H-pyrazolo[1,2-a]pyrazol-1-one, 2-amino-3-(pyrrolidin-1-yl)-6,7-dihydro-1H,5H-pyrazolo[1,2-a]pyrazol-1-one, 4,5-diamino-1,2-dimethyl-1,2-dihydropyrazol-3-one, 4,5-diamino-1,2-diethyl-1,2-dihydropyrazol-3-one, 4,5-diamino-1,2-bis(2-hydroxyethyl)-1,2-dihydropyrazol-3-one, 2-amino-3-(2-hydroxyethyl)amino-6,7-dihydro-1H,5H-pyrazolo[1,2-a]pyrazol-1-one, 2-amino-3-dimethylamino-6,7-dihydro-1H,5H-pyrazolo[1,2-a]pyrazol-1-one, 2,3-diamino-5,6,7,8-tetrahydro-1H,6H-pyridazino[1,2-a]pyrazol-1-one, 4-amino-1,2-diethyl-5-(pyrrolidin-1-yl)-1,2-dihydropyrazol-3-one, 4-amino-5-(3-dimethylaminopyrrolidin-1-yl)-1,2-diethyl-1,2-dihydropyrazol-3-one, 2,3-diamino-6-hydroxy-6,7-dihydro-1H,5H-pyrazolo[1,2-a]pyrazol-1-one, the salts thereof, the solvates thereof and/or solvates of the salts thereof.

Use will preferably be made of 2,3-diamino-6,7-dihydro-1H,5H-pyrazolo[1,2-a]pyrazol-1-one and/or a salt thereof, a solvate thereof, or a solvate of the salts thereof.

Heterocyclic bases that will preferentially be used include 4,5-diamino-1-(β-hydroxyethyl)pyrazole and/or 2,3-diamino-6,7-dihydro-1H,5H-pyrazolo[1,2-a]pyrazol-1-one and/or 2-(3-aminopyrazolo[1,5-a]pyridin-2-yl)oxyethanol and/or a salt thereof, solvates thereof or solvates of the salts thereof.

Preferably, the oxidation base(s) are chosen from para-phenylenediamines, bis(phenyl)alkylenediamines, para-aminophenols, ortho-aminophenols, heterocyclic bases, and the corresponding addition salts, the solvates thereof and/or the solvates of the salts thereof, and mixtures thereof; more preferentially from para-aminophenol, 2,3-diaminodihydropyrazolopyralozone, 2-methoxymethyl-para-phenylenediamine, 2-β-hydroxyethyl-para-phenylenediamine, 2-γ-hydroxypropyl-para-phenylenediamine, the addition salts thereof, the solvates thereof, the solvates of the salts thereof, and mixtures thereof.

The oxidation dye(s) may also comprise one or more couplers, which may be chosen from the couplers conventionally used for the dyeing of keratin fibres.

Preferably, the cosmetic composition A and/or the dyeing composition B according to the invention comprise one or more couplers.

Preferably, the couplers are chosen from meta-phenylenediamines, meta-aminophenols, meta-diphenols, naphthalene-based couplers and heterocyclic couplers, and also the addition salts thereof, and/or the solvates thereof, and/or the solvates of the salts thereof, and mixtures thereof.

Examples that may be mentioned include 1,3-dihydroxybenzene, 1,3-dihydroxy-2-methylbenzene, 4-chloro-1,3-dihydroxybenzene, 1-hydroxy-3-aminobenzene, 1-methyl-2-hydroxy-4-β-hydroxyethylaminobenzene, 4-amino-2-hydroxytoluene, 5-amino-6-chloro-2-methylphenol, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, 3-ureidoaniline, 3-ureido-1-dimethylaminobenzene, sesamol, 1-β-hydroxyethylamino-3,4-methylenedioxybenzene, α-naphthol, 2-methyl-1-naphthol, 6-hydroxyindole, 4-hydroxyindole, 4-hydroxy-N-methylindole, 5-methoxy-6-hydroxyindole, 2-amino-3-hydroxypyridine, 6-hydroxybenzomorpholine, 2-amino-4-hydroxyethylaminoanisole, 3-amino-6-methoxy-2-methylaminopyridine, 3,5-diamino-2,6-dimethoxypyridine, 1-N-(β-hydroxyethyl)amino-3,4-methylenedioxybenzene, 2,6-bis(β-hydroxyethylamino)toluene, 6-hydroxyindoline, 2,6-dihydroxy-4-methylpyridine, 2-chloro-3,5-diaminopyridine, 2-chloro-3,5-diamino-6-methoxypyridine, 2-chloro-3,5-diamino-6-methylpyridine, 1-H-3-methylpyrazol-5-one, 1-phenyl-3-methylpyrazol-5-one, 4-(3,5-diaminopyridin-2-yl)-1-(2-hydroxyethyl)-1-methylpiperazin-1-ium chloride, 2,6-dimethylpyrazolo[1,5-b]-1,2,4-triazole, 2,4,6-trimethoxyaniline hydrochlorate, 2,6-dimethyl[3,2-c]-1,2,4-triazole, 6-methylpyrazolo[1,5-a]benzimidazole and 2,6-diaminopyrazine, the addition salts thereof and/or the solvates thereof and/or the solvates of the salts thereof, and mixtures thereof.

Preferably, the coupler(s) used in the invention are chosen from 1,3-dihydroxybenzene, 1,3-dihydroxy-2-methylbenzene, 4-chloro-1,3-dihydroxybenzene, 1-hydroxy-3-aminobenzene, 1-methyl-2-hydroxy-4-β-hydroxyethylaminobenzene, 4-amino-2-hydroxytoluene, 5-amino-6-chloro-2-methylphenol, 2,4-diamino-1-(β-hydroxyethyloxy)benzene, α-naphthol, 6-hydroxyindole, 2-amino-3-hydroxypyridine, 6-hydroxybenzomorpholine, 3-amino-6-methoxy-2-methylaminopyridine, 2-amino-4-hydroxyethylaminoanisole, hydroxyethyl-3,4-methylenedioxyaniline and 2-amino-5-ethylphenol, the addition salts thereof, the solvates thereof, the solvates of the salts thereof, and mixtures thereof.

Even more preferentially, the coupler(s) used in the invention are chosen from 3-amino-6-methoxy-2-methylaminopyridine, 6-hydroxybenzomorpholine, 2,4-diamino-1-(β-hydroxyethyloxy)benzene, 2-amino-3-hydroxypyridine, 5-amino-6-chloro-2-methylphenol, 1-methyl-2-hydroxy-4-β-hydroxyethylaminobenzene, 2-amino-4-hydroxyethylaminoanisole, hydroxyethyl-3,4-methylenedioxyaniline, 2-amino-5-ethylphenol, 1-hydroxy-3-aminobenzene and 4-amino-2-hydroxytoluene, the addition salts thereof, the solvates thereof, the solvates of the salts thereof, and mixtures thereof.

In general, the addition salts of the couplers which can be used in the context of the invention are in particular chosen from the addition salts with an acid, such as hydrochlorides, hydrobromides, sulfates, citrates, succinates, tartrates, lactates, tosylates, benzenesulfonates, phosphates and acetates, and the addition salts with a base such as sodium hydroxide, potassium hydroxide, aqueous ammonia, amines or alkanolamines.

Moreover, the solvates more particularly represent the hydrates of these couplers and/or the combination of these couplers with a linear or branched C1 to C4 alcohol such as methanol, ethanol, isopropanol or n-propanol. Preferably, the solvates are hydrates.

Preferably, the couplers which can be used in the present invention are chosen from 6-hydroxybenzomorpholine, 2,4-diamino-1-(β-hydroxyethyloxy)benzene, 2-amino-3-hydroxypyridine, 5-amino-6-chloro-2-methylphenol, 1-methyl-2-hydroxy-4-β-hydroxyethylaminobenzene, 2-amino-4-hydroxyethylaminoanisole, hydroxyethyl-3,4-methylenedioxyaniline, 2-amino-5-ethylphenol, 1-hydroxy-3-aminobenzene, the addition salts thereof, the salts thereof and/or the solvates of the salts thereof, and mixtures thereof.

Even better still, the coupler(s) is or are chosen from: 6-hydroxybenzomorpholine, the addition salts thereof, the solvates thereof, the solvates of the salts thereof, hydroxyethyl-3,4-methylenedioxyaniline, the addition salts thereof, the solvates thereof, the solvates of the salts thereof, 2-amino-5-ethylphenol, the addition salts thereof, the solvates thereof, the solvates of the salts thereof, and mixtures thereof.

Advantageously, when they are present in the cosmetic composition A and/or the dyeing composition B used in the process of the invention, the total content of the oxidation base(s) ranges from 0.0001% to 10% by weight, preferably from 0.005% to 7% by weight, more preferentially from 0.1% to 4% by weight, relative to the total weight of the composition comprising them.

Advantageously, when they are present in the cosmetic composition A and/or the dyeing composition B used in the process of the invention, the total content of the coupler(s) ranges from 0.0001% to 10% by weight, preferably from 0.005% to 7% by weight, and more preferentially from 0.01% to 4% by weight, relative to the total weight of the composition comprising them.

Advantageously, when they are present in the composition A and/or the composition B used in the process of the invention, the total content of the oxidation dye(s) ranges from 0.0001% to 10% by weight, preferably from 0.005% to 7% by weight, more preferentially from 0.01% to 4% by weight, relative to the total weight of the composition comprising them.

Advantageously, when they are present in the extemporaneous mixture applied in the process of the invention, the total content of the oxidation base(s) ranges from 0.0001% to 10% by weight, preferably from 0.005% to 7% by weight, more preferentially from 0.1% to 4% by weight, relative to the total weight of the mixture.

Advantageously, when they are present in the extemporaneous mixture applied in the process of the invention, the total content of the coupler(s) ranges from 0.0001% to 10% by weight, preferably from 0.005% to 7% by weight, more preferentially from 0.01% to 4% by weight, relative to the total weight of the mixture.

Advantageously, when they are present in the extemporaneous mixture applied in the process of the invention, the total content of the oxidation dye(s) ranges from 0.0001% to 10% by weight, preferably from 0.005% to 7% by weight, more preferentially from 0.01% to 4% by weight, relative to the total weight of the mixture.

### The dyes: direct dyes

The term "direct dye" means natural and/or synthetic dyes, other than oxidation dyes. These are dyes that will spread superficially on the fibre.

The synthetic direct dyes are, for example, chosen from the dyes conventionally used for direct dyeing, and among which mention may be made of all the aromatic and/or non-aromatic dyes that are commonly used, such as nitrobenzene, azo, hydrazono, nitro(hetero)aryl, tri(hetero)arylmethane, (poly)methine, carbonyl, azine, porphyrin, metalloporphyrin, quinone and in particular anthraquinone, indoamine and phthalocyanine direct dyes, and mixtures thereof.

Among the nitrobenzene direct dyes, mention may be made of: 1,4-diamino-2-nitrobenzene, 1-amino-2-nitro-4-β-hydroxyethylaminobenzene; 1-amino-2-nitro-4-bis(β-hydroxyethyl)aminobenzene; 1,4-bis(β-hydroxyethylamino)-2-nitrobenzene; 1-β-hydroxyethylamino-2-nitro-4-bis(β-hydroxyethylamino)benzene; 1-β-hydroxyethylamino-2-nitro-4-aminobenzene; 1-β-hydroxyethylamino-2-nitro-4-(ethyl)(β-hydroxyethyl)aminobenzene; 1-amino-3-methyl-4-β-hydroxyethylamino-6-nitrobenzene; 1-amino-2-nitro-4-β-hydroxyethylamino-5-chlorobenzene; 1,2-diamino-4-nitrobenzene; 1-amino-2-β-hydroxyethylamino-5-nitrobenzene; 1,2-bis(β-hydroxyethylamino)-4-nitrobenzene; 1-amino-2-tris(hydroxymethyl)methylamino-5-nitrobenzene; 1-hydroxy-2-amino-5-nitrobenzene; 1-hydroxy-2-amino-4-nitrobenzene; 1-hydroxy-3-nitro-4-aminobenzene; 1-hydroxy-2-amino-4,6-dinitrobenzene; 1-β-hydroxyethyloxy-2-β-hydroxyethylamino-5-nitrobenzene; 1-methoxy-2-β-hydroxyethylamino-5-nitrobenzene; 1-β-hydroxyethyloxy-3-methylamino-4-nitrobenzene; 1-β,γ-dihydroxypropyloxy-3-methylamino-4-nitrobenzene; 1-β-hydroxyethylamino-4-β,γ-dihydroxypropyloxy-2-nitrobenzene; 1-β,γ-dihydroxypropylamino-4-trifluoromethyl-2-nitrobenzene; 1-β-hydroxyethylamino-4-trifluoromethyl-2-nitrobenzene; 1-β-hydroxyethylamino-3-methyl-2-nitrobenzene; 1-β-aminoethylamino-5-methoxy-2-nitrobenzene; 1-hydroxy-2-chloro-6-ethylamino-4-nitrobenzene; 1-hydroxy-2-chloro-6-amino-4-nitrobenzene; 1-hydroxy-6-bis(β-hydroxyethyl)amino-3-nitrobenzene; 1-β-hydroxyethylamino-2-nitrobenzene; 1-hydroxy-4-β-hydroxyethylamino-3-nitrobenzene.

Among the azo direct dyes, mention may be made of: Basic Red 51, Basic Orange 31, Disperse Red 17, Acid Yellow 9, Acid Black 1, Basic Red 22, Basic Red 76, Basic Yellow 57, Acid Yellow 36, Acid Orange 7, Acid Red 33, Acid Red 35, Acid Yellow 23, Acid Orange 24, Disperse Black 9, Basic Brown 16, Basic Brown 17.

Among the hydrazono direct dyes, mention may be made of: Basic Yellow 87.

Among the nitroaryl direct dyes, mention may be made of: HC Blue 2, HC Yellow 2, HC Red 3, 4-hydroxypropylamino-3-nitrophenol, N,N'-bis(2-hydroxyethyl)-2-nitrophenylenediamine.

Among the triarylmethane direct dyes, mention may be made of: Basic Violet 1, Basic Violet 2, Basic Violet 3, Basic Violet 4, Basic Violet 14, Basic Blue 1, Basic Blue 7, Basic Blue 26, Basic Green 1, Basic Blue 77 (also known as HC Blue 15), Acid Blue 1; Acid Blue 3; Acid Blue 7, Acid Blue 9; Acid Violet 49; Acid Green 3; Acid Green 5; Acid Green 50.

Among the quinone direct dyes, mention may be made of: Disperse Red 15, Solvent Violet 13, Acid Violet 43, Disperse Violet 1, Disperse Violet 4, Disperse Blue 1, Disperse Violet 8, Disperse Blue 3, Disperse Red 11, Acid Blue 62, Disperse Blue 7, Basic Blue 22, Disperse Violet 15, Basic Blue 99, and also the following compounds: 1-N-methylmorpholiniumpropylamino-4-hydroxyanthraquinone, 1-aminopropylamino-4-methylaminoanthraquinone, 1-aminopropylaminoanthraquinone, 5-β-hydroxyethyl-1,4-diaminoanthraquinone, 2-aminoethylaminoanthraquinone, 1,4-bis(β,γ-dihydroxypropylamino)anthraquinone, Acid Blue 25, Acid Blue 43, Acid Blue 78, Acid Blue 129, Acid Blue 138, Acid Blue 140, Acid Blue 251, Acid Green 25, Acid Green 41, Acid Violet 42, Mordant Red 3, Acid Black 48, HC Blue 16.

Among the azine direct dyes, mention may be made of: Basic Blue 17, Basic Red 2.

Among the indoamine direct dyes, mention may be made of: 2-β-hydroxyethylamino-5-[bis(β-4'-hydroxyethyl)amino]anilino-1,4-benzoquinone, 2-β-hydroxyethylamino-5-(2'-methoxy-4'-amino)anilino-1,4-benzoquinone, 3-N-(2'-chloro-4'-hydroxy)phenylacetylamino-6-methoxy-1,4-benzoquinoneimine, 3-N-(3'-chloro-4'-methylamino)phenylureido-6-methyl-1,4-benzoquinoneimine, 3-[4'-N-(ethylcarbamylmethyl)amino]phenylureido-6-methyl-1,4-benzoquinoneimine.

The natural direct dyes are chosen, for example, from lawsone, juglone, indigo, leuco indigo, indirubin, isatin, hennotannic acid, alizarin, carthamine, morin, purpurin, carminic acid, kermesic acid, laccaic acid, purpurogallin, protocatechaldehyde, curcumin, spinulosin, apigenidin, orceins, carotenoids, betanin, chlorophylls, chlorophyllines, monascus, polyphenols or ortho-diphenols.

Among the ortho-diphenols that are useful according to the invention, mention may be made of: catechin, quercetin, brazilin, haematein, haematoxylin, chlorogenic acid, caffeic acid, gallic acid, L-DOPA, cyanidin, (-)-epicatechin, (-)-epigallocatechin, (-)-epigallocatechin 3-gallate (EGCG), isoquercetin, pomiferin, esculetin, 6,7-dihydroxy-3-(3-hydroxy-2,4-dimethoxyphenyl)coumarin, santalin A and B, mangiferin, butein, maritimetin, sulfuretin, robtein, betanidin, pericampylinone A, theaflavin, proanthocyanidin A2, proanthocyanidin B2, proanthocyanidin C1, procyanidins DP 4-8, tannic acid, purpurogallin, 5,6-dihydroxy-2-methyl-1,4-naphthoquinone, alizarin, wedelolactone and natural extracts containing same.

Advantageously, when they are present in the cosmetic composition A and/or the dyeing composition B used in the process of the invention, the content of the direct dye(s) ranges from 0.001% to 20% by weight, preferably from 0.005% to 15% by weight, more preferentially from 0.01% to 10% by weight, better still from 0.05% to 5%, even better still from 0.1% to 3% by weight, relative to the total weight of the composition comprising them.

Advantageously, when they are present in the extemporaneous mixture applied in the process according to the invention, the total content of the direct dye(s) ranges from 0.001% to 20% by weight, preferably from 0.005% to 15% by weight, more preferentially from 0.01% to 10% by weight, better still from 0.05% to 5%, even better still from 0.1% to 3% by weight, relative to the total weight of the mixture.

### Chemical oxidizing agents

When the cosmetic composition A and/or the dyeing composition B comprises one or more oxidation dyes, the extemporaneous mixture applied in the process according to the invention also comprises one or more chemical oxidizing agents; said chemical oxidizing agent(s) being included in the dyeing composition B and/or in an oxidizing composition C. In other words, the oxidizing composition C is different from the compositions A and B.

When said oxidizing agent(s) are present in the dyeing composition B, then said dyeing composition B preferably itself results from the mixing of a composition comprising one or more alkaline agents and one or more dyes, as defined above, and of an additional oxidizing composition comprising one or more chemical oxidizing agents.

The term "chemical oxidizing agent" is understood to mean, within the meaning of the present invention, an oxidizing agent other than atmospheric oxygen.

The chemical oxidizing agent(s) which can be used in the present invention can be chosen from hydrogen peroxide, urea peroxide, alkali metal bromates, persalts, such as perborates and persulfates, in particular sodium persulfate, potassium persulfate and ammonium persulfate, peracids and oxidase enzymes (with their optional cofactors), among which mention may be made of peroxidases, 2-electron oxidoreductases, such as uricases, and 4-electron oxygenases, such as laccases, and mixtures thereof; more preferentially, the chemical oxidizing agent(s) are chosen from hydrogen peroxide, persalts and mixtures thereof, more preferably hydrogen peroxide.

Preferably, when they are present in the dyeing composition B and/or the oxidizing composition C according to the invention, the total content of the chemical oxidizing agent(s) ranges from 0.1% to 35% by weight, more preferentially from 0.5% to 25% by weight, even more preferentially from 1% to 15% by weight, relative to the weight of the composition comprising them.

According to a preferred embodiment, the chemical oxidizing agent(s) chosen from hydrogen peroxide, persalts and mixtures thereof are present in a total content ranging from 0.1% to 35% by weight, more preferentially from 0.5% to 25% by weight, more preferentially still from 1% to 15% by weight, relative to the weight of the composition comprising them.

Preferably, when they are present in the extemporaneous mixture applied in the process according to the invention, the total content of the chemical oxidizing agent(s) ranges from 0.1% to 35% by weight, more preferentially from 0.5% to 25% by weight, even more preferentially from 1% to 15% by weight, relative to the weight of the mixture.

According to one preferred embodiment, the chemical oxidizing agent(s) chosen from hydrogen peroxide, persalts and mixtures thereof, preferably hydrogen peroxide, are present, in the extemporaneous mixture applied in the process according to the invention, in a total content ranging from 0.1% to 35% by weight, more preferentially from 0.5% to 25% by weight, even more preferentially from 1% to 15% by weight, relative to the weight of the mixture.

### Cationic polymers

The cosmetic composition A, the dyeing composition B and/or the oxidizing composition C used in the process according to the invention may optionally also comprise one or more cationic polymers.

Preferably, when they are present, the cationic polymer(s) are present in the cosmetic composition A.

The cationic polymer(s) which can be used can be chosen from associative cationic polymers and non-associative cationic polymers, and mixtures thereof.

For the purposes of the present invention, the term "cationic polymer" means any polymer comprising cationic groups and/or groups that may be ionized to cationic groups. The preferred cationic polymers are chosen from those that contain units including primary, secondary, tertiary and/or quaternary amine groups that may either form part of the main polymer chain or may be borne by a side substituent directly connected thereto.

Preferably, the cationic polymers according to the invention do not comprise any anionic groups or any groups that can be ionized into an anionic group.

The cationic polymers which can be used preferably have a weight-average molar mass (Mw) of between 500 and 5×10⁶ approximately and preferably between 10³ and 3×10⁶ approximately.

Among the non-associative cationic polymers, mention may be made more particularly of:
(1) homopolymers or copolymers derived from acrylic or methacrylic esters or amides and including at least one of the units having the following formulae: 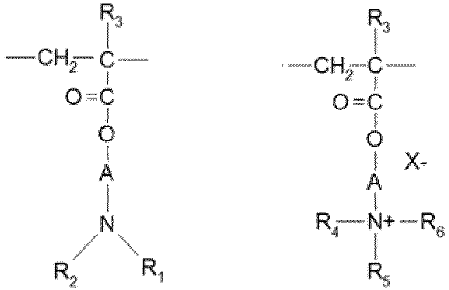 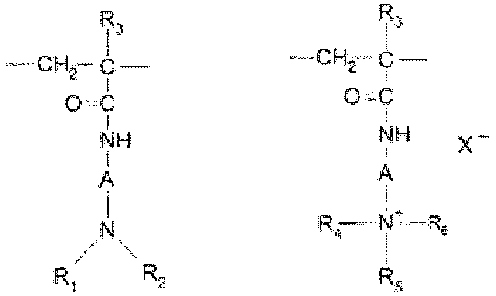 in which formulae:
   - R₃, which may be identical or different, denote a hydrogen atom or a CH₃ radical;
   - A, which may be identical or different, represent a linear or branched divalent alkyl group of 1 to 6 carbon atoms, preferably 2 or 3 carbon atoms, or a hydroxyalkyl group of 1 to 4 carbon atoms;
   - R₄, R₅ and R₆, which may be identical or different, represent an alkyl group containing from 1 to 18 carbon atoms or a benzyl radical, and preferably an alkyl group containing from 1 to 6 carbon atoms;
   - R₁ and R₂, which may be identical or different, represent a hydrogen atom or an alkyl group containing from 1 to 6 carbon atoms, preferably methyl or ethyl; and
   - X denotes an anion derived from a mineral or organic acid, such as a methosulfate anion or a halide such as chloride or bromide.

The copolymers of family (1) may also contain one or more units derived from comonomers which may be chosen from the family of acrylamides, methacrylamides, diacetone acrylamides, acrylamides and methacrylamides substituted on the nitrogen with lower (C₁-C₄) alkyls, acrylic acids or methacrylic acids or esters thereof, vinyllactams such as vinylpyrrolidone or vinylcaprolactam, and vinyl esters.

Among these copolymers of family (1), mention may be made of:
- copolymers of acrylamide and of dimethylaminoethyl methacrylate quaternized with dimethyl sulfate or with a dimethyl halide, such as the product sold under the name Hercofloc by Hercules,
- copolymers of acrylamide and of methacryloyloxyethyltrimethylammonium chloride, such as the products sold under the name Bina Quat P 100 by Ciba Geigy,
- the copolymer of acrylamide and of methacryloyloxyethyltrimethylammonium methosulfate, such as the product sold under the name Reten by Hercules,
- quaternized or non-quaternized vinylpyrrolidone/dialkylaminoalkyl acrylate or methacrylate copolymers, such as the products sold under the name Gafquat by ISP, for instance Gafquat 734 or Gafquat 755, or alternatively the products known as Copolymer 845, 958 and 937. These polymers are described in detail in French patents 2077 143 and 2 393 573,
- dimethylaminoethyl methacrylate/vinylcaprolactam/vinylpyrrolidone terpolymers, such as the product sold under the name Gaffix VC 713 by ISP,
- vinylpyrrolidone/methacrylamidopropyldimethylamine copolymers, such as the products sold under the name Styleze CC 10 by ISP;
- quaternized vinylpyrrolidone/dimethylaminopropylmethacrylamide copolymers such as the product sold under the name Gafquat HS 100 by ISP;
- polymers, preferably crosslinked polymers, of methacryloyloxy(C₁-C₄)alkyltri(C₁-C₄)alkylammonium salts, such as the polymers obtained by homopolymerization of dimethylaminoethyl methacrylate quaternized with methyl chloride, or by copolymerization of acrylamide with dimethylaminoethyl methacrylate quaternized with methyl chloride, the homo- or copolymerization being followed by crosslinking with an olefinically unsaturated compound, in particular methylenebisacrylamide. Use may be made more particularly of a crosslinked acrylamide/methacryloyloxyethyltrimethylammonium chloride copolymer (20/80 by weight) in the form of a dispersion comprising 50% by weight of said copolymer in mineral oil. This dispersion is sold under the name Salcare^{®} SC 92 by Ciba. Use may also be made of a crosslinked methacryloyloxyethyltrimethylammonium chloride homopolymer comprising approximately 50% by weight of the homopolymer in mineral oil or in a liquid ester. These dispersions are sold under the names Salcare^{®} SC 95 and Salcare^{®} SC 96 by Ciba;

(2) cationic polysaccharides, notably cationic celluloses and galactomannan gums. Among the cationic polysaccharides, mention may be made more particularly of cellulose ether derivatives including quaternary ammonium groups, cationic cellulose copolymers or cellulose derivatives grafted with a water-soluble quaternary ammonium monomer and cationic galactomannan gums.

The cellulose ether derivatives including quaternary ammonium groups are notably described in FR 1 492 597, and mention may be made of the polymers sold under the name Ucare Polymer JR (JR 400 LT, JR 125 and JR 30M) or LR (LR 400 and LR 30M) by Amerchol. These polymers are also defined in the CTFA dictionary as quaternary ammoniums of hydroxyethyl cellulose that have reacted with an epoxide substituted with a trimethylammonium group.

Cationic cellulose copolymers or cellulose derivatives grafted with a water-soluble quaternary ammonium monomer are described notably in patent US 4 131 576, and mention may be made of hydroxyalkyl celluloses, for instance hydroxymethyl, hydroxyethyl or hydroxypropyl celluloses notably grafted with a methacryloylethyltrimethylammonium, methacrylamido-propyltrimethylammonium or dimethyldiallylammonium salt. The commercial products corresponding to this definition are more particularly the products sold under the names Celquat L 200 and Celquat H 100 by National Starch.

The cationic galactomannan gums are described more particularly in patents US 3 589 578 and US 4 031 307, and mention may be made of guar gums comprising cationic trialkylammonium groups. Use is made, for example, of guar gums modified with a 2,3-epoxypropyltrimethylammonium salt (for example, a chloride). Such products are notably sold under the names Jaguar C13 S, Jaguar C 15, Jaguar C 17 and Jaguar C162 by Rhodia;

(3) polymers formed from piperazinyl units and divalent alkylene or hydroxyalkylene radicals containing linear or branched chains, optionally interrupted with oxygen, sulfur or nitrogen atoms or with aromatic or heterocyclic rings, and also the oxidation and/or quaternization products of these polymers;

(4) water-soluble polyaminoamides prepared in particular by polycondensation of an acidic compound with a polyamine; these polyaminoamides can be crosslinked with an epihalohydrin, a diepoxide, a dianhydride, an unsaturated dianhydride, a bis-unsaturated derivative, a bis-halohydrin, a bis-azetidinium, a bis-haloacyldiamine, a bis-alkyl halide or alternatively with an oligomer resulting from the reaction of a difunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent being used in proportions ranging from 0.025 to 0.35 mol per amine group of the polyamino amide; these polyaminoamides can be alkylated or, if they include one or more tertiary amine functions, they can be quaternized;

(5) polyaminoamide derivatives resulting from the condensation of polyalkylene polyamines with polycarboxylic acids followed by alkylation with difunctional agents. Mention may be made, for example, of adipic acid/dialkylaminohydroxyalkyldialkylenetriamine polymers in which the alkyl radical includes from 1 to 4 carbon atoms and preferably denotes methyl, ethyl or propyl. Among these derivatives, mention may be made more particularly of the adipic acid/dimethylaminohydroxypropyl/diethylenetriamine polymers sold under the name Cartaretine F, F4 or F8 by Sandoz;

(6) polymers obtained by reacting a polyalkylene polyamine including two primary amine groups and at least one secondary amine group with a dicarboxylic acid chosen from diglycolic acid and saturated aliphatic dicarboxylic acids containing from 3 to 8 carbon atoms; the mole ratio between the polyalkylene polyamine and the dicarboxylic acid preferably being between 0.8:1 and 1.4:1; the resulting polyaminoamide being reacted with epichlorohydrin in a mole ratio of epichlorohydrin relative to the secondary amine group of the polyaminoamide preferably of between 0.5:1 and 1.8:1. Polymers of this type are sold in particular under the name Hercosett 57 by Hercules Inc. or under the name PD 170 or Delsette 101 by Hercules in the case of the adipic acid/epoxypropyl/diethylenetriamine copolymer;

(7) cyclopolymers of alkyldiallylamine or of dialkyldiallylammonium, such as homopolymers or copolymers including, as main constituent of the chain, units corresponding to formula (VI) or (VII): 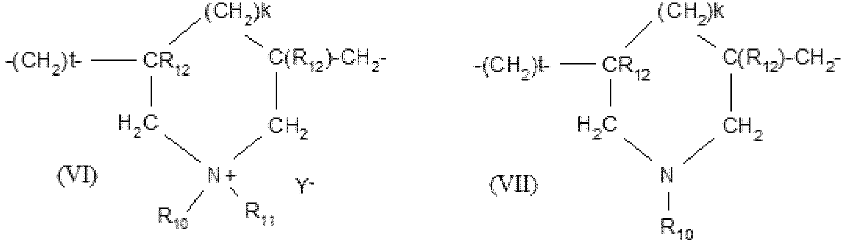 in which formulae (VI) and (VII):
- k and t are equal to 0 or 1, the sum k + t being equal to 1;
- R₁₂ denotes a hydrogen atom or a methyl radical;
- R₁₀ and R₁₁, independently of one another, denote an alkyl group containing from 1 to 6 carbon atoms, a hydroxyalkyl group in which the alkyl group contains 1 to 5 carbon atoms, a C₁-C₄ amidoalkyl group; or alternatively R₁₀ and R₁₁ may denote, together with the nitrogen atom to which they are attached, heterocyclic groups such as piperidinyl or morpholinyl; R₁₀ and R₁₁, independently of one another, preferably denote an alkyl group containing from 1 to 4 carbon atoms; and
- Y⁻ is an anion such as bromide, chloride, acetate, borate, citrate, tartrate, bisulfate, bisulfite, sulfate or phosphate.

Mention may be made more particularly of the dimethyldiallylammonium salt (for example chloride) homopolymer sold, for example, under the name Merquat 100 by Nalco (and homologues thereof of low weight-average molar masses) and the copolymers of diallyldimethylammonium salts (for example chloride) and of acrylamide, notably sold under the names Merquat 550 and Merquat 7SPR;

(8) quaternary diammonium polymers comprising repeating units of formula: 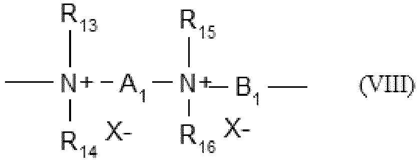 in which formula (VIII):
- R₁₃, R₁₄, R₁₅ and R₁₆, which may be identical or different, represent aliphatic, alicyclic or arylaliphatic radicals containing from 1 to 20 carbon atoms or lower hydroxyalkylaliphatic radicals, or alternatively R₁₃, R₁₄, R₁₅ and R₁₆, together or separately, constitute, with the nitrogen atoms to which they are attached, heterocycles optionally comprising a second non-nitrogen heteroatom, or alternatively R₁₃, R₁₄, R₁₅ and R₁₆ represent a linear or branched C₁ to C₆ alkyl radical substituted with a nitrile, ester, acyl or amide group or a group -CO-O-R₁₇-D or -CO-NH-R₁₇-D where R is an alkylene and D is a quaternary ammonium group;
- A₁ and B₁ represent divalent polymethylene groups comprising from 2 to 20 carbon atoms which may be linear or branched, and saturated or unsaturated, and which may contain, linked to or inserted in the main chain, one or more aromatic rings, or one or more oxygen or sulfur atoms or sulfoxide, sulfone, disulfide, amino, alkylamino, hydroxyl, quaternary ammonium, ureido, amide or ester groups; and
- X⁻ denotes an anion derived from a mineral or organic acid;

it being understood that A₁, R₁₃ and R₁₅ can form, with the two nitrogen atoms to which they are attached, a piperazine ring;
in addition, if A₁ denotes a linear or branched, saturated or unsaturated alkylene or hydroxyalkylene radical, B₁ can also denote a group (CH₂)ₙ-CO-D-OC-(CH₂)ₙ- in which D denotes:
   a) a glycol residue of formula -O-Z-O-, in which Z denotes a linear or branched hydrocarbon radical or a group corresponding to one of the following formulae: -(CH₂-CH₂-O)ₓ-CH₂-CH₂- and -[CH₂CH(CH₃)-O]_{y}-CH₂-CH(CH₃)-, where x and y denote an integer from 1 to 4, representing a defined and unique degree of polymerization or any number from 1 to 4 representing an average degree of polymerization;
   b) a bis-secondary diamine residue, such as a piperazine derivative;
   c) a bis-primary diamine residue of formula: -NH-Y-NH-, where Y denotes a linear or branched hydrocarbon radical, or alternatively the divalent radical -CH₂-CH₂-S-S-CH₂-CH₂-; or
   d) a ureylene group of formula: -NH-CO-NH-.

Preferably, X⁻ is an anion, such as chloride or bromide. These polymers have a number-average molar mass (Mn) generally of between 1000 and 100 000.

Mention may be made more particularly of polymers which are constituted of repeating units corresponding to the formula: 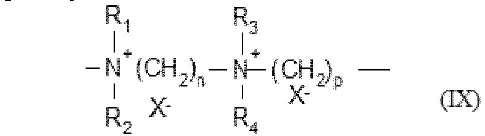 in which formula (IX) R₁, R₂, R₃ and R₄, which may be identical or different, denote an alkyl or hydroxyalkyl radical containing from 1 to 4 carbon atoms approximately, n and p are integers ranging from 2 to 20 approximately, and X⁻ is an anion derived from a mineral or organic acid.

A compound of formula (IX) that is particularly preferred is the one for which R₁, R₂, R₃ and R₄ represent a methyl radical and n = 3, p = 6 and X = Cl, which is known as Hexadimethrine chloride according to the INCI (CTFA) nomenclature;
(9) polyquaternary ammonium polymers comprising units of formula (X): 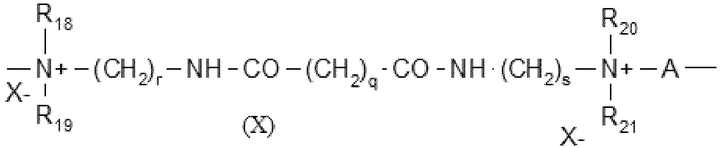 in which formula (X):
- R₁₈, R₁₉, R₂₀ and R₂₁, which may be identical or different, represent a hydrogen atom or a methyl, ethyl, propyl, β-hydroxyethyl, β-hydroxypropyl or - CH₂CH₂(OCH₂CH₂)ₚOH radical, where p is equal to 0 or to an integer of between 1 and 6, with the proviso that R₁₈, R₁₉, R₂₀ and R₂₁ do not simultaneously represent a hydrogen atom,
- r and s, which may be identical or different, are integers between 1 and 6,
- q is equal to 0 or to an integer between 1 and 34,
- X⁻ denotes an anion, such as a halide, and
- A denotes a dihalide radical or preferably represents -CH₂-CH₂-O-CH₂-CH₂-

Examples that may be mentioned include the products Mirapol^{®} A 15, Mirapol^{®} AD1, Mirapol^{®} AZ1 and Mirapol^{®} 175 sold by Miranol;
(10) quaternary polymers of vinylpyrrolidone and of vinylimidazole, for instance the products sold under the names Luviquat^{®} FC 905, FC 550 and FC 370 by BASF;
(11) polyamines such as Polyquart^{®} H sold by Cognis, which is referenced under the name Polyethylene Glycol (15) Tallow Polyamine in the CTFA dictionary;
(12) polymers including in their structure:
   (a) one or more units corresponding to formula (A) below: 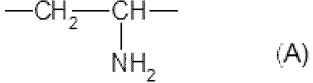
   (b) optionally one or more units corresponding to formula (B) below:

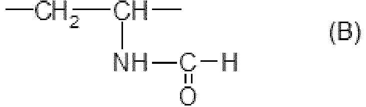

In other words, these polymers may be notably chosen from homopolymers or copolymers including one or more units derived from vinylamine and optionally one or more units derived from vinylformamide.

Preferably, these cationic polymers are chosen from polymers including, in their structure, from 5 mol% to 100 mol% of units corresponding to formula (A) and from 0 to 95 mol% of units corresponding to formula (B), preferentially from 10 mol% to 100 mol% of units corresponding to formula (A) and from 0 to 90 mol% of units corresponding to formula (B).

These polymers may be obtained, for example, by partial hydrolysis of polyvinylformamide. This hydrolysis may take place in acidic or basic medium.

The weight-average molecular mass of said polymer, measured by light scattering, may range from 1000 to 3 000 000 g/mol, preferably from 10 000 to 1 000 000 and more particularly from 100 000 to 500 000 g/mol.

The cationic charge density of these polymers may range from 2 meq/g to 20 meq/g, preferably from 2.5 to 15 meq/g and more particularly from 3.5 to 10 meq/g.

The polymers including units of formula (A) and optionally units of formula (B) are notably sold under the name Lupamin by BASF, for instance, in a non-limiting manner, the products sold under the names Lupamin 9095, Lupamin 5095, Lupamin 1095, Lupamin 9030 (or Luviquat 9030) and Lupamin 9010.

The cationic polymers can also be chosen from associative cationic polymers.

It is recalled that "associative polymers" are polymers that are capable, in an aqueous medium, of reversibly associating with one another or with other molecules.

Their chemical structure more particularly comprises at least one hydrophilic zone and at least one hydrophobic zone.

The term "hydrophobic group" means a radical or polymer with a saturated or unsaturated, linear or branched hydrocarbon chain, comprising at least 8 carbon atoms, preferably from 8 to 30 carbon atoms, in particular from 12 to 30 carbon atoms.

Preferentially, the hydrocarbon group is derived from a monofunctional compound. By way of example, the hydrophobic group may be derived from a fatty alcohol such as stearyl alcohol, cetyl alcohol, dodecyl alcohol or decyl alcohol. It may also denote a hydrocarbon polymer, for instance polybutadiene.

Among the associative cationic polymers which can be used, mention may be made, alone or as a mixture, of:
- (A) cationic associative polyurethanes, which can be represented by general formula (Ia) below:

   R-X-(P)ₙ-[L-(Y)ₘ]ᵣ- L'-(P')ₚ-X'-R'

   in which:
   R and R', which may be identical or different, represent a hydrophobic group or a hydrogen atom;
   X and X', which may be identical or different, represent a group comprising an amine function optionally bearing a hydrophobic group, or alternatively a group L";
   L, L' and L", which may be identical or different, represent a group derived from a diisocyanate;
   P and P', which may be identical or different, represent a group including an amine function optionally bearing a hydrophobic group;
   Y represents a hydrophilic group;
   r is an integer between 1 and 100 inclusive, preferably between 1 and 50 inclusive and in particular between 1 and 25 inclusive;
   n, m and p are each, independently of one another, between 0 and 1000 inclusive;
   the molecule containing at least one protonated or quaternized amine function and at least one hydrophobic group.

Preferably, the only hydrophobic groups are the groups R and R' at the chain ends.

One preferred family of cationic associative polyurethanes is the one corresponding to formula (Ia) described above, in which:
R and R' both independently represent a hydrophobic group,
X and X' each represent a group L",
n and p are integers that are between 1 and 1000 inclusive, and L, L', L", P, P', Y and m have the meaning indicated above.

Another preferred family of cationic associative polyurethanes is the one corresponding to formula (Ia) above in which:
- n = p = 0 (the polymers do not include any units derived from a monomer containing an amine function, incorporated into the polymer during the polycondensation),
- the protonated amine functions result from the hydrolysis of excess isocyanate functions, at the chain end, followed by alkylation of the primary amine functions formed with alkylating agents containing a hydrophobic group, i.e. compounds of the type RQ or R'Q, in which R and R' are as defined above and Q denotes a leaving group such as a halide or a sulfate.

Yet another preferred family of cationic associative polyurethanes is the one corresponding to formula (Ia) above in which:
R and R' both independently represent a hydrophobic group,
X and X' both independently represent a group including a quaternary amine,
n = p = 0, and
L, L', Y and m have the meaning given above.

The number-average molecular mass (Mn) of the cationic associative polyurethanes is preferably between 400 and 500 000 inclusive, in particular between 1000 and 400 000 inclusive and ideally between 1000 and 300 000 inclusive.

The term "hydrophobic group" means a radical or polymer containing a saturated or unsaturated, linear or branched hydrocarbon chain, which may contain one or more heteroatoms such as P, O, N or S, or a radical containing a perfluoro or silicone chain. When the hydrophobic group denotes a hydrocarbon radical, it includes at least 8 carbon atoms, preferably from 8 to 30 carbon atoms, in particular from 12 to 30 carbon atoms and more preferentially from 18 to 30 carbon atoms.

Preferentially, the hydrocarbon group is derived from a monofunctional compound.

By way of example, the hydrophobic group may be derived from a fatty alcohol such as stearyl alcohol, cetyl alcohol, dodecyl alcohol or decyl alcohol. It may also denote a hydrocarbon polymer, for instance polybutadiene.

When X and/or X' denote(s) a group including a tertiary or quaternary amine, X and/or X' may represent one of the following formulae: 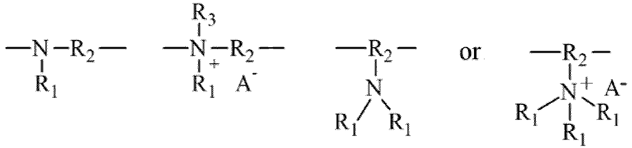 for X 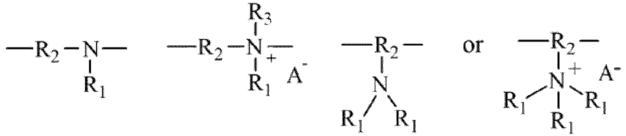 for X' in which:
R₂ represents a linear or branched alkylene radical containing from 1 to 20 carbon atoms, optionally including a saturated or unsaturated ring, or an arylene radical, one or more of the carbon atoms possibly being replaced with a heteroatom chosen from N, S, O and P;
R₁ and R₃, which may be identical or different, denote a linear or branched C1-C30 alkyl or alkenyl radical or an aryl radical, at least one of the carbon atoms possibly being replaced with a heteroatom chosen from N, S, O and P;
A⁻ is a physiologically acceptable anionic counterion such as a halide, for instance chloride or bromide, or mesylate.

The groups L, L' and L" represent a group of formula: 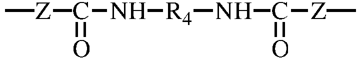 in which:
Z represents -O-, -S- or -NH-; and
R₄ represents a linear or branched alkylene radical containing from 1 to 20 carbon atoms, optionally including a saturated or unsaturated ring, or an arylene radical, one or more of the carbon atoms possibly being replaced with a heteroatom chosen from N, S, O and P.

The groups P and P' comprising an amine function may represent at least one of the following formulae: 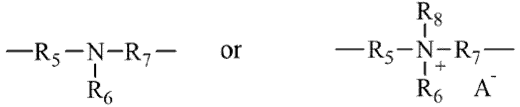 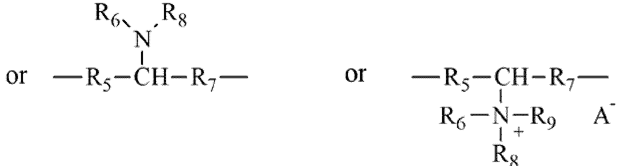 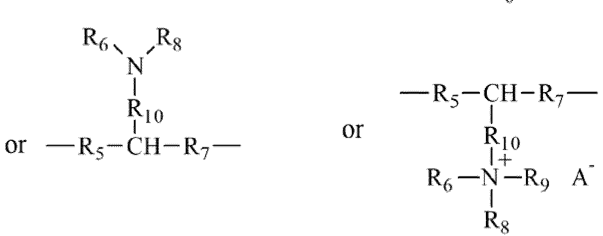 in which:
R₅ and R₇ have the same meanings as R₂ defined previously;
R₆, R₈ and R₉ have the same meanings as R₁ and R₃ defined previously;
R₁₀ represents a linear or branched, optionally unsaturated alkylene group possibly containing one or more heteroatoms chosen from N, O, S and P; and
A⁻ is a physiologically acceptable anionic counterion such as a halide, for instance chloride or bromide, or mesylate.
As regards the meaning of Y, the term "hydrophilic group" means a polymeric or non-polymeric water-soluble group.
By way of example, when it is not a polymer, mention may be made of ethylene glycol, diethylene glycol and propylene glycol.

When it is a hydrophilic polymer, in accordance with one preferred embodiment, mention may be made, for example, of polyethers, sulfonated polyesters, sulfonated polyamides or a mixture of these polymers. The hydrophilic compound is preferentially a polyether and notably a poly(ethylene oxide) or poly(propylene oxide).

The cationic associative polyurethanes of formula (Ia) according to the invention are formed from diisocyanates and from various compounds bearing functions containing labile hydrogen. The functions containing labile hydrogen may be alcohol, primary or secondary amine or thiol functions, giving, after reaction with the diisocyanate functions, polyurethanes, polyureas and polythioureas, respectively. In the present invention, the term "polyurethanes" encompasses these three types of polymer, namely polyurethanes per se, polyureas and polythioureas, and also copolymers thereof.

A first type of compound involved in the preparation of the polyurethane of formula (Ia) is a compound including at least one unit bearing an amine function. This compound may be multifunctional, but the compound is preferentially difunctional, that is to say that, according to a preferential embodiment, this compound includes two labile hydrogen atoms borne, for example, by a hydroxyl, primary amine, secondary amine or thiol function. A mixture of multifunctional and difunctional compounds in which the percentage of multifunctional compounds is low may also be used.

As mentioned above, this compound may include more than one unit containing an amine function. In this case, it is a polymer bearing a repetition of the unit containing an amine function.

Compounds of this type may be represented by one of the following formulae:
HZ-(P)ₙ-ZH or HZ-(P')ₚ-ZH, in which Z, P, P', n and p are as defined above.

Examples that may be mentioned include N-methyldiethanolamine, N-tert-butyldiethanolamine and N-sulfoethyldiethanolamine.

The second compound included in the preparation of the polyurethane of formula (XIV) is a diisocyanate corresponding to the formula:
O=C=N-R₄-N=C=O in which R₄ is as defined above.

By way of example, mention may be made of methylenediphenyl diisocyanate, methylenecyclohexane diisocyanate, isophorone diisocyanate, tolylene diisocyanate, naphthalene diisocyanate, butane diisocyanate and hexane diisocyanate.

A third compound involved in the preparation of the polyurethane of formula (Ia) is a hydrophobic compound intended to form the terminal hydrophobic groups of the polymer of formula (Ia).

This compound is constituted of a hydrophobic group and a function containing a labile hydrogen, for example a hydroxyl, primary or secondary amine, or thiol function.

By way of example, this compound may be a fatty alcohol such as stearyl alcohol, dodecyl alcohol or decyl alcohol. When this compound includes a polymeric chain, it may be, for example, α-hydroxylated hydrogenated polybutadiene.

The hydrophobic group of the polyurethane of formula (Ia) may also result from the quaternization reaction of the tertiary amine of the compound including at least one tertiary amine unit. Thus, the hydrophobic group is introduced via the quaternizing agent. This quaternizing agent is a compound of the type RQ or R'Q, in which R and R' are as defined above and Q denotes a leaving group such as a halide, a sulfate, etc.

The cationic associative polyurethane may also comprise a hydrophilic block. This block is provided by a fourth type of compound involved in the preparation of the polymer. This compound may be multifunctional. It is preferably difunctional. It is also possible to have a mixture in which the percentage of multifunctional compound is low.

The functions containing labile hydrogen are alcohol, primary or secondary amine or thiol functions. This compound may be a polymer terminated at the chain ends with one of these functions containing labile hydrogen.

By way of example, when it is not a polymer, mention may be made of ethylene glycol, diethylene glycol and propylene glycol.

When it is a hydrophilic polymer, mention may be made, for example, of polyethers, sulfonated polyesters and sulfonated polyamides, or a mixture of these polymers. The hydrophilic compound is preferentially a polyether and notably a poly(ethylene oxide) or poly(propylene oxide).

The hydrophilic group termed Y in formula (Ia) is optional. Specifically, the units containing a quaternary or protonated amine function may suffice to provide the solubility or water-dispersibility required for this type of polymer in an aqueous solution.

Although the presence of a hydrophilic group Y is optional, cationic associative polyurethanes including such a group are, however, preferred.
- (B) quaternized cellulose derivatives, and in particular quaternized celluloses modified with groups comprising at least one fatty chain, such as linear or branched alkyl groups, linear or branched arylalkyl groups, or linear or branched alkylaryl groups, preferably linear or branched alkyl groups, these groups comprising at least 8 carbon atoms, in particular from 8 to 30 carbon atoms, better still from 10 to 24, or even from 10 to 14, carbon atoms; or mixtures thereof.

Preferably, mention may be made of quaternized hydroxyethylcelluloses modified with groups including at least one fatty chain, such as linear or branched alkyl groups, linear or branched arylalkyl groups, or linear or branched alkylaryl groups, preferably linear or branched alkyl groups, these groups including at least 8 carbon atoms, notably from 8 to 30 carbon atoms, better still from 10 to 24 or even from 10 to 14 carbon atoms; or mixtures thereof.

Preferentially, mention may be made of the hydroxyethylcelluloses of formula (Ib): 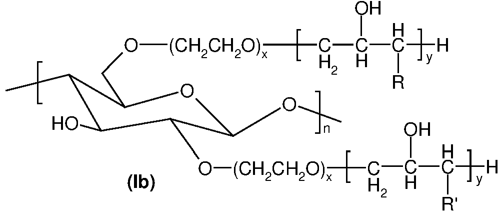 in which:
- R represents an ammonium group RaRbRcN⁺-, Q⁻ in which Ra, Rb and Rc, which may be identical or different, represent a hydrogen atom or a linear or branched C₁-C₃₀ alkyl, and Q⁻ represents an anionic counterion such as a halide, for instance a chloride or bromide; preferably an alkyl;
- R' represents an ammonium group R'aR'bR'cN⁺-, Q'⁻ in which R'a, R'b and R'c, which may be identical or different, represent a hydrogen atom or a linear or branched C₁-C₃₀ alkyl, and Q'⁻ represents an anionic counterion such as a halide, for instance a chloride or bromide; preferably an alkyl;
   it being understood that at least one of the radicals Ra, Rb, Rc, R'a, R'b and R'c represents a linear or branched C₈-C₃₀ alkyl;
- n, x and y, which may be identical or different, represent an integer of between 1 and 10 000.

Preferably, in formula (Ib), at least one of the radicals Ra, Rb, Rc, R'a, R'b and R'c represents a linear or branched C₈-C₃₀, better still C₁₀-C₂₄, or even C₁₀-C₁₄, alkyl; mention may be made in particular of the dodecyl radical (C12). Preferably, the other radical(s) represent a linear or branched C₁-C₄ alkyl, in particular methyl.

Preferably, in formula (Ib), only one of the radicals Ra, Rb, Rc, R'a, R'b and R'c represents a linear or branched C₈-C₃₀, better still C₁₀-C₂₄, or even C₁₀-C₁₄, alkyl; mention may be made in particular of the dodecyl radical (C12). Preferably, the other radicals represent a linear or branched C₁-C₄ alkyl, in particular methyl.

Even better still, R can be a group chosen from -N⁺(CH₃)₃ Q'⁻ and - N⁺(C₁₂H₂₅)(CH₃)₂ Q'⁻, preferably an -N⁺(CH₃)₃ Q'⁻ group.

Even better still, R' may be a group -N⁺(C₁₂H₂₅)(CH₃)₂, Q'⁻.

The aryl radicals preferably denote phenyl, benzyl, naphthyl or anthryl groups.

Mention may notably be made of the polymers having the following INCI names:
- Polyquaternium-24, such as the product Quatrisoft LM 200^{®}, sold by Amerchol/Dow Chemical;
- PG-Hydroxyethylcellulose Cocodimonium Chloride, such as the product Crodacel QM^{®};
- PG-Hydroxyethylcellulose Lauryldimonium Chloride (C12 alkyl), such as the product Crodacel QL^{®}; and
- PG-Hydroxyethylcellulose Stearyldimonium Chloride (C18 alkyl), such as the product Crodacel QS^{®}, sold by Croda.

Mention may also be made of the hydroxyethylcelluloses of formula (Ib) in which R represents a trimethylammonium halide and R' represents a dimethyldodecylammonium halide, preferentially R represents trimethylammonium chloride (CH₃)₃N⁺-, Cl⁻ and R' represents dimethyldodecylammonium chloride (CH₃)₂(C₁₂H₂₅)N⁺-, Cl⁻. This type of polymer is known under the INCI name Polyquaternium-67; as commercial products, mention may be made of the Softcat Polymer SL^{®} polymers, such as SL-100, SL-60, SL-30 and SL-5, from Amerchol/Dow Chemical.

More particularly, the polymers of formula (Ib) are, for example, those the viscosity of which is between 2000 and 3000 cPs inclusive, preferentially between 2700 and 2800 cPs. Typically, Softcat Polymer SL-5 has a viscosity of 2500 cPs, Softcat Polymer SL-30 has a viscosity of 2700 cPs, Softcat Polymer SL-60 has a viscosity of 2700 cPs and Softcat Polymer SL-100 has a viscosity of 2800 cPs. Use may also be made of Softcat Polymer SX-1300X with a viscosity of between 1000 and 2000 cPs.
- (C) cationic polyvinyllactams, in particular those comprising:
   - a) at least one monomer of vinyllactam or alkylvinyllactam type;
   - b) at least one monomer of structure (Ic) or (IIc) below: 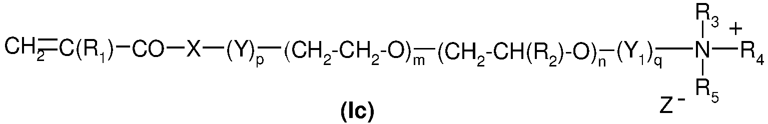 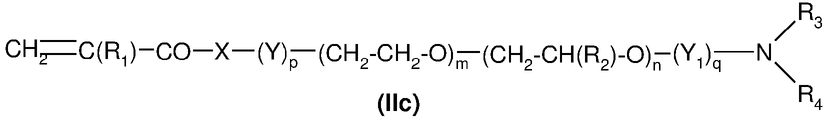 in which:
      - X denotes an oxygen atom or an NR6 radical,
      - R₁ and R₆ denote, independently of one another, a hydrogen atom or a linear or branched C₁-C₅ alkyl radical,
      - R₂ denotes a linear or branched C₁-C₄ alkyl radical,
      - R₃, R₄ and R₅ denote, independently of one another, a hydrogen atom, a linear or branched C₁-C₃₀ alkyl radical or a radical of formula (IIIc):

         -(Y₂)ᵣ-(CH₂-CH(R₇)-O)ₓ-R₈ **(IIIC)**
      - Y, Y₁ and Y₂ denote, independently of one another, a linear or branched C₂-C₁₆ alkylene radical,
      - R₇ denotes a hydrogen atom, or a linear or branched C₁-C₄ alkyl radical or a linear or branched C₁-C₄ hydroxyalkyl radical,
      - R₈ denotes a hydrogen atom or a linear or branched C₁-C₃₀ alkyl radical,
      - p, q and r denote, independently of one another, 0 or 1,
      - m and n denote, independently of one another, an integer ranging from 0 to 100 inclusive,
      - x denotes an integer ranging from 1 to 100 inclusive,
      - Z denotes an anionic counterion of an organic or mineral acid, such as a halide, for instance chloride or bromide, or mesylate;
      with the proviso that:
      - at least one of the substituents R₃, R₄, R₅ or R₈ denotes a linear or branched C₈-C₃₀ alkyl radical,
      - if m and/or n is other than zero, then q is equal to 1,
      - if m = n = 0, then p or q is equal to 0.

The cationic poly(vinyllactam) polymers according to the invention may be crosslinked or non-crosslinked and may also be block polymers.

Preferably, the counterion Z- of the monomers of formula (Ic) is chosen from halide ions, phosphate ions, the methosulfate ion and the tosylate ion.

Preferably, R₃, R₄ and R₅ denote, independently of one another, a hydrogen atom or a linear or branched C₁-C₃₀ alkyl radical.

More preferentially, the monomer b) is a monomer of formula (Ic) for which, preferentially, m and n are equal to 0.

The vinyllactam or alkylvinyllactam monomer is preferably a compound of structure (IVc): 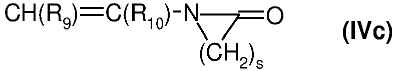 in which:
- s denotes an integer ranging from 3 to 6,
- R₉ denotes a hydrogen atom or a linear or branched C₁-C₅ alkyl radical,
- R₁₀ denotes a hydrogen atom or a linear or branched C₁-C₅ alkyl radical,
with the proviso that at least one of the radicals R9 and R10 denotes a hydrogen atom.

Even more preferentially, the monomer (IVc) is vinylpyrrolidone.

The cationic poly(vinyllactam) polymers according to the invention may also contain one or more additional monomers, preferably cationic or non-ionic monomers.

As compounds that are particularly preferred, mention may be made of the following terpolymers comprising at least:
a) one monomer of formula (IVc),
b) one monomer of formula (Ic) in which p=1, m=n=q=0, R₃ and R₄ denote, independently of one another, a hydrogen atom or a C₁-C₅ alkyl radical and R₅ denotes a linear or branched C₈-C₂₄ alkyl radical, and
c) one monomer of formula (IIc) in which p=1, m=n=q=0, R₃ and R₄ denote, independently of one another, a hydrogen atom or a linear or branched C₁-C₅ alkyl radical.

Even more preferentially, terpolymers comprising, by weight, 40% to 95% of monomer (a), 0.1% to 55% of monomer (c) and 0.25% to 50% of monomer (b) will be used. Such polymers are notably described in patent application WO-00/68282.

As cationic polymer(vinyllactam) polymers according to the invention, the following are notably used:
- vinylpyrrolidone/ dimethylaminopropylmethacrylamide/ dodecyldimethylmethacrylamidopropylammonium tosylate terpolymers,
- vinylpyrrolidone/ dimethylaminopropylmethacrylamide/ cocoyldimethylmethacrylamidopropylammonium tosylate terpolymers,
- vinylpyrrolidone/ dimethylaminopropylmethacrylamide/ lauryldimethylmethacrylamidopropylammonium tosylate or chloride terpolymers.

The vinylpyrrolidone/ dimethylaminopropylmethacrylamide/ lauryldimethylmethacrylamidopropylammonium chloride terpolymer is notably sold by ISP under the names Styleze W10^{®} and Styleze W20L^{®} (INCI name: Polyquaternium-55).

The weight-average molecular mass (Mw) of the cationic poly(vinyllactam) polymers is preferably between 500 and 20 000 000, more particularly between 200 000 and 2 000 000 and preferentially between 400 000 and 800 000.
- (D) the cationic polymers obtained by polymerization of a monomer mixture comprising one or more vinyl monomers substituted with one or more amino groups, one or more hydrophobic non-ionic vinyl monomers, and one or more associative vinyl monomers, as described in patent application WO 2004/024779.

Among these copolymers, mention may be made more particularly of the products of polymerization of a monomer mixture comprising:
- a di(C₁-C₄ alkyl)amino(C₁-C₆ alkyl) methacrylate,
- one or more C₁-C₃₀ alkyl esters of (meth)acrylic acid,
- a polyethoxylated C10-C30 alkyl methacrylate (20-25 mol of ethylene oxide unit),
- a 30/5 polyethylene glycol/polypropylene glycol allyl ether,
- a hydroxy(C₂-C₆ alkyl) methacrylate, and
- an ethylene glycol dimethacrylate.

Such a polymer is, for example, the compound sold by Lubrizol under the name Carbopol Aqua CC^{®} and which corresponds to the INCI name Polyacrylate-1 Crosspolymer.

Preferably, when they are present in the cosmetic composition A, the dyeing composition B and/or the oxidizing composition C used in the process according to the invention, the cationic polymer(s) are chosen from non-associative cationic polymers and mixtures thereof, more preferentially from cationic polysaccharides, alkyldiallylamine cyclopolymers or dialkyldiallylammonium cyclopolymers and mixtures thereof, and even better still from guar gums, dimethyldiallylammonium salt homopolymers, copolymers of salts of diallyldimethylammonium and of acrylamide, and mixtures thereof.

When they are present in the cosmetic composition A, the dyeing composition B and/or the optional oxidizing composition C, the total content of the cationic polymer(s) preferably ranges from 0.01% to 10% by weight, more preferentially from 0.05% to 5% by weight, even more preferentially from 0.1% to 2% by weight, relative to the total weight of the composition comprising them.

### Non-ionic surfactants

The cosmetic composition A, the dyeing composition B and the optional oxidizing composition C used in the process according to the invention may optionally also comprise one or more non-ionic surfactants.

Examples of non-ionic surfactants which can be used in the compositions of the present invention are described, for example, in the Handbook of Surfactants by M.R. Porter, published by Blackie & Son (Glasgow and London), 1991, pages 116-178. They are chosen in particular from alcohols, α-diols, (C₁-C₂₀)alkylphenols or acids which are fatty, these compounds being polyethoxylated, polypropoxylated or polyglycerolated and having at least one fatty chain comprising, for example, from 8 to 18 carbon atoms, it being possible for the number of ethylene oxide or propylene oxide groups to range in particular from 1 to 100 and it being possible for the number of glycerol groups to range in particular from 1 to 30.

Mention may be made, as examples of non-ionic surfactants which can be used according to the present invention, of the following non-ionic surfactants:
- oxyalkylenated (C₈-C₂₄)alkylphenols;
- saturated or unsaturated, linear or branched, oxyalkylenated or glycerolated C₈ to C₄₀ alcohols;
- saturated or unsaturated, linear or branched, oxyalkylenated C₈-C₃₀ fatty acid amides;
- esters of saturated or unsaturated, linear or branched, C₈ to C₃₀ acids and of polyols or of polyethylene glycols;
- esters of saturated or unsaturated, linear or branched, C₈ to C₃₀ acids and of sorbitol or of sorbitan which are preferably oxyethylenated;
- fatty acid esters of sucrose;
- (C₈-C₃₀)alkyl(poly)glucosides, (C₈-C₃₀)alkenyl(poly)glucosides, which are optionally oxyalkylenated (0 to 10 oxyalkylene units) and which comprise from 1 to 15 glucose units, esters of (C₈-C₃₀)alkyl(poly)glucosides;
- saturated or unsaturated oxyethylenated plant oils;
- condensates of ethylene oxide and/or of propylene oxide;
- N-(C₈-C₃₀)alkylglucamine and N-(C₈-C₃₀)acylmethylglucamine derivatives;
- aldobionamides;
- amine oxides;
- oxyethylenated and/or oxypropylenated silicones;
- and mixtures thereof.

The oxyalkylene units are more particularly oxyethylene or oxypropylene units, or a combination thereof, preferably oxyethylene units.

The number of moles of ethylene oxide and/or of propylene oxide preferably ranges from 1 to 250, more particularly from 2 to 100 and better still from 2 to 70; the number of moles of glycerol ranges in particular from 1 to 50 and better still from 1 to 10.

Advantageously, the non-ionic surfactants according to the invention do not comprise oxypropylene units.

Use is preferably made, as examples of glycerolated non-ionic surfactants, of mono- or polyglycerolated C₈ to C₄₀ alcohols, comprising from 1 to 50 mol of glycerol, preferably from 1 to 10 mol of glycerol.

Among the glycerolated alcohols, it is more particularly preferred to use the C₈/C₁₀ alcohol having 1 mol of glycerol, the C₁₀/C₁₂ alcohol having 1 mol of glycerol and the C₁₂ alcohol having 1.5 mol of glycerol.

Preference is particularly given, among esters of saturated or unsaturated, linear or branched, C₈ to C₃₀ acids and of sorbitol, to esters of saturated or unsaturated, linear or branched, C₈ to C₃₀ fatty acids and of sorbitan, comprising from 1 to 20 oxyethylene units, and more preferentially to esters of C₈ to C₁₈ fatty acid and of sorbitan, comprising from 4 to 20 oxyethylene units, better still esters of saturated or unsaturated, linear, C₈ to C₁₈ fatty acids and of sorbitan, comprising from 4 to 20 oxyethylene units.

Such compounds are known in particular under the name of polysorbates. They are, inter alia, sold under the name Tween by Uniqema. Mention may, for example, be made of polyoxyethylene (4) sorbitan monolaurate (polysorbate 21), sold under the name Tween 21, polyoxyethylene (20) sorbitan monolaurate (polysorbate 20), sold under the name Tween 20, polyoxyethylene (20) sorbitan monopalmitate (polysorbate 40), sold under the name Tween 40, polyoxyethylene (20) sorbitan monostearate (polysorbate 60), sold under the name Tween 60, polyoxyethylene (4) sorbitan monostearate (polysorbate 61), sold under the name Tween 61, polyoxyethylene (20) sorbitan tristearate (polysorbate 65), sold under the name Tween 65, polyoxyethylene (20) sorbitan monooleate (polysorbate 80), sold under the name Tween 80, polyoxyethylene (5) sorbitan monooleate (polysorbate 81), sold under the name Tween 81, and polyoxyethylene (20) sorbitan trioleate (polysorbate 85), sold under the name Tween 85.

The non-ionic surfactant(s) which can be used in the present invention are preferentially chosen from:
- oxyethylenated C₈ to C₄₀ alcohols comprising from 1 to 100 mol of ethylene oxide, preferably from 2 to 50 and more particularly from 2 to 40 mol of ethylene oxide;
- saturated or unsaturated oxyethylenated plant oils comprising from 1 to 100, preferably from 2 to 70, mol of ethylene oxide;
- and mixtures thereof.

When they are present in the cosmetic composition A, the dyeing composition B and/or the optional oxidizing composition C used in the process according to the invention, the total content of the non-ionic surfactant(s) preferably ranges from 0.05% to 10% by weight, more preferentially from 0.1% to 8% by weight, better still from 0.5% to 5% by weight, relative to the total weight of the composition comprising them.

When they are present in the extemporaneous mixture applied in the process according to the invention, the total content of the non-ionic surfactant(s) ranges, preferably, from 0.05% to 10% by weight, more preferentially from 0.1% to 8% by weight, better still from 0.5% to 5% by weight, relative to the total weight of the mixture.

### Anionic surfactants

The cosmetic composition A, the dyeing composition B and/or the optional oxidizing composition C used in the process according to the invention may optionally also comprise one or more anionic surfactants.

The term "anionic surfactant" is understood to mean a surfactant including, as ionic or ionizable groups, only anionic groups. These anionic groups are preferably chosen from the following groups: CO₂H, CO₂⁻, SO₃H, SO₃⁻, OSO₃H, OSO₃⁻, H₂PO₃, HPO₃⁻, PO₃²⁻, H₂PO₂, HPO₂⁻, PO₂²⁻, POH and PO⁻.

Preferably, when they are present, the anionic surfactant(s) are chosen from sulfate-type anionic surfactants.

For the purposes of the present invention, the term "anionic surfactant of sulfate type" is intended to mean an anionic surfactant comprising one or more sulfate functions (-OSO₃H or -OSO₃⁻).

Such surfactants may advantageously be chosen from alkyl sulfates, alkyl ether sulfates, alkylamido sulfates, alkylamido ether sulfates, alkylaryl polyether sulfates, monoglyceride sulfates; and salts thereof and mixtures thereof; the alkyl groups of these compounds comprising in particular from 8 to 30 carbon atoms, preferably from 8 to 26, and more preferentially from 10 to 22 carbon atoms; the aryl group preferably denoting a phenyl or benzyl group; it being possible for these compounds to be polyoxyalkylenated, in particular polyoxyethylenated, and then preferably comprising from 1 to 50 ethylene oxide units, and more preferentially from 2 to 10 ethylene oxide units.

Preferably, the sulfate-type anionic surfactant(s) are chosen from:
- alkyl sulfates, notably C8 to C26 and preferably C10 to C22 alkyl sulfates;
- alkyl ether sulfates, notably C₈ to C₂₆ and preferably C₁₀ to C₂₂ alkyl ether sulfates, preferably comprising from 1 to 10 ethylene oxide units; and
- mixtures thereof.

When the anionic surfactant(s) of sulfate type are in salt form, said salt may be chosen from alkali metal salts, such as the sodium or potassium salt, ammonium salts, amine salts and in particular amino alcohol salts, and alkaline earth metal salts, such as the magnesium salt, and mixtures thereof.

Examples of amino alcohol salts that may be mentioned include monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxymethyl)aminomethane salts.

Alkali metal or alkaline-earth metal salts and in particular sodium or magnesium salts are preferably used.

Preferably, the sulfate-type anionic surfactant(s) are chosen from (C₁₀-C₂₂) alkyl sulfates, more preferentially from sodium, triethanolamine, magnesium or ammonium (C₁₀-C₂₂) alkyl sulfates, sodium, ammonium or magnesium (C₁₀-C₂₂) alkyl ether sulfates, and mixtures thereof.

Even better still, the sulfate-type anionic surfactant(s) are chosen from (C₁₀-C₂₂) alkyl sulfates having the INCI name sodium lauryl sulfate, such as the compound sold under the name Texapon Z95P by BASF, or having the INCI name sodium cetearyl sulfate, such as the product sold under the name Lanette E by BASF.

Advantageously, when they are present in the composition according to the invention, the anionic surfactant(s) are chosen from sulfate-type anionic surfactants, preferably from (C₁₀-C₂₂) alkyl sulfates, preferentially sodium lauryl sulfate, sodium cetearyl sulfate, and mixtures thereof, better still sodium cetearyl sulfate.

Preferably, when they are present in the cosmetic composition A, the dyeing composition B and/or the optional oxidizing composition C, the total content of the anionic surfactant(s) ranges from 0.5% to 10% by weight, more preferentially from 0.8% to 5% by weight, even more preferentially from 1% to 3% by weight, relative to the total weight of the composition comprising them.

In one particular embodiment, the total content of sulfate-type anionic surfactants, present in the cosmetic composition A, the dyeing composition B and/or the optional oxidizing composition C. preferably ranges from 0.5% to 10% by weight, more preferentially from 0.8% to 5% by weight, even more preferentially from 1% to 3% by weight, relative to the total weight of the composition comprising them.

### Non-associative anionic acrylic polymers

The cosmetic composition A, the dyeing composition B and/or the optional oxidizing composition C used in the process according to the invention may optionally also comprise one or more non-associative anionic acrylic polymers.

Among the non-associative anionic acrylic polymers that can be used according to the invention, mention may be made of crosslinked acrylic acid or methacrylic acid homopolymers or copolymers, crosslinked 2-acrylamido-2-methylpropanesulfonic acid homopolymers and crosslinked acrylamide copolymers thereof, ammonium acrylate homopolymers, or copolymers of ammonium acrylate and of acrylamide, alone or as mixtures.

A first family of non-associative anionic acrylic polymers that is suitable for use is represented by crosslinked (meth)acrylic acid homopolymers, in particular crosslinked acrylic acid homopolymers.

Among the homopolymers of this type, mention may be made of those crosslinked with an allyl alcohol ether of the sugar series, for instance the products sold under the names Carbopol 980, 981, 954, 2984 and 5984 by Noveon or the products sold under the names Synthalen M and Synthalen K by 3 VSA. These polymers have the INCI name Carbomer.

The non-associative anionic acrylic polymers may also be crosslinked (meth)acrylic acid copolymers, such as the polymers sold under the names Carbopol Aqua SF1 and Carbopol Aqua SF2 by Noveon.

The non-associative anionic acrylic polymers may be chosen from crosslinked 2-acrylamido-2-methylpropanesulfonic acid homopolymers and the crosslinked acrylamide copolymers thereof.

Among the partially or totally neutralized crosslinked copolymers of 2-acrylamido-2-methylpropanesulfonic acid and of acrylamide, mention may be made in particular of the product described in Example 1 of EP 503 853, and reference may be made to said document as regards these polymers.

The composition may similarly comprise, as non-associative anionic acrylic polymers, ammonium acrylate homopolymers or copolymers of ammonium acrylate and of acrylamide.

As examples of ammonium acrylate homopolymers, mention may be made of the product sold under the name Microsap PAS 5193 by Hoechst. Among the copolymers of ammonium acrylate and of acrylamide, mention may be made of the product sold under the name Bozepol C Nouveau or the product PAS 5193 sold by Hoechst. Reference may notably be made to FR 2 416 723, US 2 798 053 and US 2 923 692 as regards the description and preparation of such compounds.

When they are present, the non-associative anionic acrylic polymer(s) are preferably chosen from crosslinked (meth)acrylic acid homopolymers or copolymers, more preferentially from crosslinked (meth)acrylic acid homopolymers.

When they are present in the cosmetic composition A, the dyeing composition B and/or the optional oxidizing composition C, the total content of the non-associative anionic acrylic polymer(s) preferably ranges from 0.05% to 10% by weight, more preferentially from 0.1% to 8% by weight, even more preferentially from 0.5% to 5% by weight, relative to the total weight of the composition comprising them.

### Solvents

The cosmetic composition A, the dyeing composition B and/or the optional oxidizing composition C can also comprise at least one organic solvent, other than propane-1,3-diol described above.

Examples of organic solvents that may be mentioned include linear or branched C₂-C₄ alkanols, such as ethanol, propanol and isopropanol; polyols other than propane-1,3-diol and polyol ethers, for instance 2-butoxyethanol, propylene glycol, glycerol, dipropylene glycol, propylene glycol monomethyl ether, diethylene glycol monomethyl ether and monoethyl ether, and also aromatic alcohols or ethers, for instance benzyl alcohol or phenoxyethanol, and mixtures thereof.

Preferably, the organic solvent(s) is/are chosen from polyols; more preferentially, the organic solvent is glycerol.

When they are present in the cosmetic composition A, the dyeing composition B and/or the optional oxidizing composition C used in the process of the invention, the total content of the organic solvent(s), other than propane-1,3-diol advantageously ranges from 0.01% to 30% by weight, preferably ranging from 2% to 25% by weight, relative to the total weight of the composition comprising them.

In addition, the cosmetic composition A, the dyeing composition B and/or the optional oxidizing composition C are preferably aqueous compositions. Preferably, the cosmetic composition A, the dyeing composition B and/or the optional oxidizing composition C comprise water in an amount of greater than or equal to 5% by weight, preferably of greater than or equal to 10% by weight, better still of greater than or equal to 15% by weight, relative to the total weight of the composition which comprises it.

Preferably, the water content ranges from 15% to 95% by weight, preferentially from 30% to 92% by weight and better still from 40% to 90% by weight, relative to the total weight of the composition which comprises said water.

### Additives

The cosmetic composition A, the dyeing composition B and/or the optional oxidizing composition C, used in the process according to the invention, can also optionally comprise one or more additional compounds different from the compounds defined above, preferably chosen from cationic surfactants, amphoteric or zwitterionic surfactants, reducing agents, emollients, anti-foams, moisturising agents, UV-screening agents, peptizers, solubilizers, fragrances, anionic, non-ionic or amphoteric polymers or mixtures thereof, antidandruff agents, antiseborrhoeic agents, vitamins and pro-vitamins, including panthenol, sunscreens, plasticizers, solubilizing agents other than the solvents of the invention, acidifying agents, mineral or organic thickeners, in particular polymeric thickeners, antioxidants, hydroxy acids, preserving agents, and mixtures thereof.

Preferably, when the additional compound(s) above are present in the cosmetic composition A, the dyeing composition B and/or the optional oxidizing composition C, the additional compound(s) are generally present in a content of, for each of them, between 0.01% and 20% by weight, relative to the total weight of the composition comprising them.

Of course, those skilled in the art will take care to choose this or these optional additional compounds in a way such that the advantageous properties intrinsically attached to the cosmetic composition of the invention are not, or not substantially, detrimentally affected by the envisaged addition(s).

### Process

The present invention relates to a process for dyeing keratin fibres, in particular human keratin fibres such as the hair, comprising the application to said keratin fibres of an extemporaneous mixture (that is to say a mixture prepared at the time of use):
i) of at least one composition A comprising:
   - propane-1,3-diol, present in a total content of greater than or equal to 3% by weight, relative to the total weight of the composition A,
   - one or more alkaline agents, and
   - one or more dyes chosen from oxidation dyes, direct dyes and mixtures thereof; and
ii) of at least one composition B comprising:
   - one or more alkaline agents, and
   - one or more dyes chosen from oxidation dyes, direct dyes and mixtures thereof;

it being understood that, when the composition A and/or B comprise at least one oxidation dye, the extemporaneous mixture also comprises one or more oxidizing agents; said oxidizing agent(s) being present in the composition B and/or in an oxidizing composition C.

In other words, the process of the invention comprises a first step of mixing the compositions A and B and optionally oxidizing composition C, directly followed by the application of the resulting mixture to the keratin fibres.

When said oxidizing agent(s) are present in the dyeing composition B, then said dyeing composition B preferably itself results from the mixing of a composition comprising one or more alkaline agents and one or more dyes and of an oxidizing composition comprising one or more chemical oxidizing agents. In other words, according to this embodiment, the process of the invention comprises a prior step of preparing the composition B, before the step of mixing the compositions A and B.

According to a first embodiment, the dye(s) included in the compositions A and B of the process are chosen from direct dyes and mixtures thereof. According to this embodiment, the process of the invention comprises the application to said keratin fibres of an extemporaneous mixture:
i) of at least one composition A comprising:
   - propane-1,3-diol, present in a total content of greater than or equal to 3% by weight, relative to the total weight of the composition A,
   - one or more alkaline agents, and
   - one or more dyes chosen from direct dyes and mixtures thereof; and
ii) of at least one composition B comprising:
   - one or more alkaline agents,
   - one or more dyes chosen from direct dyes and mixtures thereof.

According to a second embodiment, the dye(s) included in the compositions A and B of the process are chosen from oxidation dyes, direct dyes and mixtures thereof, preferably oxidation dyes and mixtures thereof, and the chemical oxidizing agent(s) are included in the composition B. According to this embodiment, the composition B is preferably prepared beforehand by mixing at least two compositions before it is mixed with the composition A.

According to a first variant of this embodiment, the process of the invention comprises the application to said keratin fibres of an extemporaneous mixture:
i) of at least one composition A comprising:
   - propane-1,3-diol, present in a total content of greater than or equal to 3% by weight, relative to the total weight of the composition A,
   - one or more alkaline agents, and
   - one or more dyes chosen from oxidation dyes, direct dyes and mixtures thereof; and
ii) of at least one composition B resulting from the mixing:
   - of a composition B' comprising:
      - one or more alkaline agents,
      - one or more dyes chosen from oxidation dyes, direct dyes and mixtures thereof, and
   - of an oxidizing composition comprising:
      - one or more chemical oxidizing agents.

According to a second variant of this embodiment, the process of the invention comprises the application to said keratin fibres of an extemporaneous mixture:
i) of at least one composition A comprising:
   - propane-1,3-diol, present in a total content of greater than or equal to 3% by weight, relative to the total weight of the composition A,
   - one or more alkaline agents, and
   - one or more dyes chosen from direct dyes and mixtures thereof;
ii) of at least one composition B resulting from the mixing:
   - of a composition B' comprising:
      - one or more alkaline agents,
      - one or more dyes chosen from oxidation dyes and mixtures thereof, and
      - optionally, one or more dyes chosen from direct dyes and mixtures thereof; and
   - of an oxidizing composition comprising:
      - one or more chemical oxidizing agents.

According to yet a third variant of this particular embodiment, the process of the invention comprises the application to said keratin fibres of an extemporaneous mixture:
i) of at least one composition A comprising:
   - propane-1,3-diol, present in a total content of greater than or equal to 3% by weight, relative to the total weight of the composition A,
   - one or more alkaline agents, and
   - one or more dyes chosen from oxidation dyes and mixtures thereof, and
   - optionally, one or more dyes chosen from direct dyes and mixtures thereof; and
ii) of at least one composition B resulting from the mixing:
   - of a composition B' comprising:
      - one or more alkaline agents,
      - one or more dyes chosen from oxidation dyes and mixtures thereof, and
      - optionally, one or more dyes chosen from direct dyes and mixtures thereof; and
   - of an oxidizing composition comprising:
      - one or more chemical oxidizing agents.

According to yet a fourth variant of this particular embodiment, the process of the invention comprises the application to said keratin fibres of an extemporaneous mixture:
i) of at least one composition A comprising:
   - propane-1,3-diol, present in a total content of greater than or equal to 3% by weight, relative to the total weight of the composition A,
   - one or more alkaline agents, and
   - one or more dyes chosen from oxidation dyes and mixtures thereof, and
   - optionally, one or more dyes chosen from direct dyes and mixtures thereof; and
ii) of at least one composition B resulting from the mixing:
   - of a composition B' comprising:
      - one or more alkaline agents,
      - one or more dyes chosen from direct dyes and mixtures thereof, and
   - of an oxidizing composition comprising:
      - one or more chemical oxidizing agents.

In other words, according to these four variants of this second embodiment, the chemical oxidizing agent(s) are included in the dyeing composition B which results from the mixing of a composition B' comprising one or more alkaline agents and one or more dyes and of an oxidizing composition C comprising one or more chemical oxidizing agents.

In this embodiment, the weight ratio between the composition A and the composition B (that is to say of the mixture of the composition B' and the oxidizing composition), in the extemporaneous mixture applied in the process of the invention, preferably ranges from 0.01 to 10, more preferentially from 0.05 to 5, better still from 0.075 to 1.

According to yet a fifth variant of this particular embodiment, the process of the invention comprises the application to said keratin fibres of an extemporaneous mixture:
i) of at least one composition A comprising:
   - propane-1,3-diol, present in a total content of greater than or equal to 3% by weight, relative to the total weight of the composition A,
   - one or more alkaline agents, and
   - one or more dyes chosen from oxidation dyes and mixtures thereof, and
   - optionally, one or more dyes chosen from direct dyes and mixtures thereof; and
ii) of at least one composition B comprising:
   - one or more alkaline agents,
   - one or more dyes chosen from direct dyes and mixtures thereof, and
   - one or more chemical oxidizing agents.

According to yet a third embodiment, the dye(s) included in the compositions A and B of the process are chosen from oxidation dyes, direct dyes and mixtures thereof, preferably oxidation dyes and mixtures thereof, and the chemical oxidizing agent(s) are included in an oxidizing composition C, other than the compositions A and B.

According to a first variant of this other embodiment, the process of the invention comprises the application to said keratin fibres of an extemporaneous mixture:
i) of at least one composition A comprising:
   - propane-1,3-diol, present in a total content of greater than or equal to 3% by weight, relative to the total weight of the composition A,
   - one or more alkaline agents, and
   - one or more dyes chosen from oxidation dyes, direct dyes and mixtures thereof;
ii) of at least one composition B comprising:
   - one or more alkaline agents,
   - one or more dyes chosen from oxidation dyes, direct dyes and mixtures thereof; and
iii) of at least one oxidizing composition C comprising one or more chemical oxidizing agents.

According to a second variant of this other particular embodiment, the process of the invention comprises the application to said keratin fibres of an extemporaneous mixture:
i) of at least one composition A comprising:
   - propane-1,3-diol, present in a total content of greater than or equal to 3% by weight, relative to the total weight of the composition A,
   - one or more alkaline agents, and
   - one or more dyes chosen from direct dyes and mixtures thereof;
ii) of at least one composition B comprising:
   - one or more alkaline agents,
   - one or more dyes chosen from oxidation dyes and mixtures thereof, and
   - optionally, one or more dyes chosen from direct dyes and mixtures thereof; and
iii) of at least one oxidizing composition C comprising one or more chemical oxidizing agents.

According to yet a third variant of this other particular embodiment, the process of the invention comprises the application to said keratin fibres of an extemporaneous mixture:
i) of at least one composition A comprising:
   - propane-1,3-diol, present in a total content of greater than or equal to 3% by weight, relative to the total weight of the composition A,
   - one or more alkaline agents, and
   - one or more dyes chosen from oxidation dyes and mixtures thereof, and
   - optionally, one or more dyes chosen from direct dyes and mixtures thereof; and
ii) of at least one composition B comprising:
   - one or more alkaline agents,
   - one or more dyes chosen from direct dyes and mixtures thereof; and
iii) of at least one oxidizing composition C comprising one or more chemical oxidizing agents.

According to a fourth variant of this other particular embodiment, the process of the invention comprises the application to said keratin fibres of an extemporaneous mixture:
i) of at least one composition A comprising:
   - propane-1,3-diol, present in a total content of greater than or equal to 3% by weight, relative to the total weight of the composition A,
   - one or more alkaline agents, and
   - one or more dyes chosen from oxidation dyes and mixtures thereof, and
   - optionally, one or more dyes chosen from direct dyes and mixtures thereof; and
ii) of at least one composition B comprising:
   - one or more alkaline agents,
   - one or more dyes chosen from oxidation dyes and mixtures thereof, and
   - optionally, one or more dyes chosen from direct dyes and mixtures thereof; and
iii) of at least one oxidizing composition C comprising one or more chemical oxidizing agents.

In other words, according to this other embodiment, the chemical oxidizing agent(s) are included in an oxidizing composition C other than the compositions A and B. Thus, the extemporaneous mixture applied in the process according to this embodiment also comprises at least one composition C comprising one or more oxidizing agents.

In this embodiment, the weight ratio between, on the one hand, the composition A and the composition B and, on the other hand, between the composition B and the composition C in the extemporaneous mixture applied in the process of the invention, preferably ranges, respectively, from 0.01 to 5 and from 0.1 to 2, preferentially from 0.1 to 2 and from 0.3 to 2, better still from 0.2 to 1 and from 0.5 to 1.

Advantageously, the composition A is a booster composition, the composition B is a dyeing composition, optionally comprising an oxidizing agent, and the composition C is an oxidizing composition. The term "booster" composition is intended to mean that the composition A, comprising propane-1,3-diol, makes it possible in particular to improve the dyeing performance of the dyeing composition B.

The extemporaneous mixture of the process according to the invention can be applied to wet or dry keratin fibres, and also to all types of fair or dark, natural or dyed, permanent-waved, bleached or relaxed, fibres.

According to one particular embodiment of the process of the invention, the fibres are washed before applying the extemporaneous mixture described above.

The application of the extemporaneous mixture to the keratin fibres may be performed by any conventional means, in particular using a comb, a fine brush, a coarse brush or with the fingers.

The bath ratio of the extemporaneous mixture applied to the keratin fibres preferably ranges from 0.1 to 10 and more preferentially from 0.2 to 5. For the purposes of the present invention, the term "bath ratio" is intended to mean the ratio between the total weight of the applied extemporaneous mixture and the total weight of keratin fibres to be treated.

The dyeing process, i.e. the mixing of the compositions A, B and optionally C, followed by the application of the resulting mixture to the keratin fibres, is generally performed at ambient temperature (between 15 and 25°C).

The extemporaneous mixture may be applied to the keratin fibres for a leave-on time ranging from 30 to 60 minutes.

On conclusion of the process, the keratin fibres can be optionally subjected to washing with a shampoo and/or can be rinsed with water, before being dried or left to dry.

### Device

The invention also relates to a multi-compartment device comprising a first compartment containing the composition A according to the invention as described above, and a second compartment containing a composition B according to the invention as described above, and optionally a third compartment containing one or more chemical oxidizing agents as described above, preferably hydrogen peroxide.

According to a first embodiment, the multi-compartment device of the invention comprises a first compartment containing the composition A according to the invention as described above, and a second compartment containing a composition B according to the invention as described above.

According to another particular embodiment, the multi-compartment device of the invention comprises a first compartment containing the composition A according to the invention as described above, a second compartment containing a composition B according to the invention, and a third compartment containing a composition C comprising one or more chemical oxidizing agents as described above, preferably hydrogen peroxide.

The examples that follow serve to illustrate the invention without, however, being limiting in nature.

### Examples

In the examples which follow, all the amounts are shown as percentage by weight of active material (AM) relative to the total weight of the composition (unless otherwise mentioned).

### Example 1:

### Booster composition

The composition A (according to the invention) and the composition A' (comparative) were prepared from the ingredients of which the contents are shown in the table below (% am):

**[Table 1]**

| | Composition A | Composition A' |
|---|---|---|
| Ethanolamine | 1.7 | 1.7 |
| Hydroxybenzomorpholine | 0.053 | 0.053 |
| Polyquaternium-6 | 0.8 | 0.8 |
| 4-Amino-2-hvdroxvtoluene | 0.041 | 0.041 |
| **Propane-1,3-diol** | **5** | - |
| **Glycerol** | - | **5** |
| Ascorbic acid | 0.4 | 0.4 |
| N,N-bis(2-hydroxyethyl)-p-phenylenediamine sulfate | 0.029 | 0.029 |
| m-Aminophenol | 0.068 | 0.068 |
| EDTA | 0.2 | 0.2 |
| 2,4-Diaminophenoxyethanol hydrochloride | 0.05 | 0.05 |
| PEG-60 hydrogenated castor oil | 1.1 | 1.1 |
| Sodium chloride | 2 | 2 |
| Sodium metabisulfite | 0.1 | 0.1 |
| 2,5-Toluenediamine | 0.168 | 0.168 |
| Carbomer | 1.96 | 1.96 |
| Fragrance | 0.4 | 0.4 |
| Water | qs 100 | qs 100 |

### Dyeing composition

The composition B was prepared from the ingredients of which the contents are shown in the table below (% am):

**[Table 2]**

| | Composition B |
|---|---|
| Resorcinol | 0.4 |
| Oleic acid | 2.7 |
| Ammonium hydroxide | 4.94 |
| Ethanolamine | 0.63 |
| m-Aminophenol | 0.04 |
| EDTA | 0.2 |
| 2,4-Diaminophenoxyethanol hydrochloride | 0.004 |
| Cetearyl alcohol | 16.2 |
| Olevl alcohol | 2.7 |
| Hexadimethrine chloride | 3 |
| Oleth-30 | 3.6 |
| Sodium metabisulfite | 0.71 |
| 2,5-Toluenediamine | 0.44 |
| Fragrance | 0.5 |
| Water | qs 100 |

### Oxidizing composition

An oxidizing composition C was prepared from the ingredients of which the contents are shown in the table below (% am):

**[Table 3]**

| | Composition C |
|---|---|
| Trideceth-2 carboxamide MEA | 0.85 |
| Tetrasodium etidronate | 0.06 |
| Sodium salicylate | 0.035 |
| Glycerol | 0.5 |
| Cetearyl alcohol | 2.28 |
| Ceteareth-25 | 0.57 |
| Phosphoric acid | q.s. pH 2 +/- 0.2 |
| Hydrogen peroxide | 6 |
| Tetrasodium pyrophosphate | 0.04 |
| Water | qs 100 |

### Dyeing protocol

At the time of use, each of the dyeing compositions A and A' is mixed with the dyeing composition B and the oxidizing composition C, in a 1/4/6 ratio. Each of the mixtures is then applied to a lock of natural hair containing 90% white hairs, in a proportion of 10 g of mixture per gram of hair.

After a leave-on time of 35 minutes on a plate thermostatically controlled at 27°C, the hair is rinsed, washed with a standard shampoo and dried.

### Results

Colorimetric measurements were taken using a Konica Minolta CM-3600A spectrocolorimeter (illuminant D65, angle 10°, specular component included) in the CIELab system.

The strength of the colourings is measured.

L* represents the lightness; the lower the value of L*, the more powerful the colouration obtained.

The results are shown in the table below:

**[Table 4]**

| | L* |
|---|---|
| A+B+C (invention) | 25.53 |
| A'+B+C (comparative) | 28.26 |

The booster composition A according to the invention (comprising propane-1,3-diol) results in a value L* that is lower compared to the composition A' (comprising glycerol). The composition A thus makes it possible to obtain a stronger colouring than the comparative composition A'.

### Example 2:

The composition A1 (according to the invention) and the composition A1' (comparative) were prepared from the ingredients of which the contents are shown in the table below (% am):

**[Table 5]**

| | Composition A1 | Composition A1' |
|---|---|---|
| Ethanolamine | 1.7 | 1.7 |
| Polyquaternium-6 | 0.8 | 0.8 |
| **Propane-1,3-diol** | **5** | - |
| **1,2-propylene glycol** | - | **5** |
| Ascorbic acid | 0.4 | 0.4 |
| 2-Nitro-5-glyceryl methvlaniline | 1 | 1 |
| 2-amino-6-chloro-4-nitrophenol | 0.5 | 0.5 |
| EDTA | 0.2 | 0.2 |
| PEG-60 hydrogenated castor oil | 1.1 | 1.1 |
| Sodium chloride | 2 | 2 |
| Sodium metabisulfite | 0.1 | 0.1 |
| Carbomer | 1.96 | 1.96 |
| Fragrance | 0.4 | 0.4 |
| Water | qs 100 | qs 100 |

The dyeing composition B and the oxidizing composition C were prepared as in Example 1 above.

### Dyeing protocol

At the time of use, each of the dyeing compositions A1 and A1' is mixed with the dyeing composition B and the oxidizing composition C, in a 1/4/6 ratio. Each of mixture is then applied to a lock of natural hair containing 90% white hairs (WN) and to a lock of permanent-waved hair (PW), in a proportion of 10 g of mixture per gram of hair.

After a leave-on time of 35 minutes on a plate thermostatically controlled at 27°C, the hair is rinsed, washed with a standard shampoo and dried.

### Results

Colorimetric measurements were taken using a Konica Minolta CM-3600A spectrocolorimeter (illuminant D65, angle 10°, specular component included) in the CIELab system.

The strength of the colourings is measured: L* represents the lightness; the lower the value of L*, the more powerful the colouration obtained.

Selectivity is represented by the color difference ΔE between the lock of colored natural hair (WN) and the lock of colored permanent-waived hair (PW). The lower the value of ΔE, the lower the selectivity, and therefore the better.

The results are shown in the table below:

**[Table 6]**

| | Lock type | L* | a* | b* | ΔE |
|---|---|---|---|---|---|
| A1 + B + C (invention) | WN | 26.02 | 4.31 | 12.35 | 5.18 |
| | PW | 21.58 | 3.96 | 9.71 | |
| A1' + B + C (comparative) | WN | 29.18 | 4.63 | 15.27 | 7.98 |
| | PW | 23.97 | 3.31 | 9.37 | |

The mixture (A1 + B + C) according to the invention leads to lower values of L*, thus to a more powerful coloration compared to the mixture (A1' + B + C) (according to the prior art).

The mixture (A1 + B + C) according to the invention has a lower ΔE value than the mixture (A1' + B + C) according to the prior art. The mixture (A1 + B + C) according to the invention leads to a lower selectivity, thus better than that of the mixture (A1' + B + C) according to the prior art.

## Claims

1. Process for dyeing keratin fibres, in particular human keratin fibres such as the hair, comprising the application to said keratin fibres of an extemporaneous mixture:
i) of at least one cosmetic composition A comprising:
- propane-1,3-diol, present in a total content of greater than or equal to 3% by weight, relative to the total weight of the composition A,
- one or more alkaline agents, and
- one or more dyes chosen from oxidation dyes, direct dyes and mixtures thereof; and
ii) of at least one dyeing composition B comprising:
- one or more alkaline agents, and
- one or more dyes chosen from oxidation dyes, direct dyes and mixtures thereof;
it being understood that, when the composition A and/or B comprise at least one oxidation dye, the extemporaneous mixture also comprises one or more oxidizing agents; said oxidizing agent(s) being present in the composition B and/or in an oxidizing composition C.

2. Process according to Claim 1, **characterized in that** the total content of propane-1,3-diol ranges from 3% to 15% by weight, preferably from 4% to 10% by weight and more preferentially from 4% to 7% by weight, relative to the total weight of the composition A.

3. Process according to either one of the preceding claims, **characterized in that** the alkaline agent(s) are chosen from alkanolamines, aqueous ammonia, alkali metal or alkaline earth metal carbonates or bicarbonates, alkali metal or alkaline earth metal silicates or metasilicates, and mixtures thereof, more preferentially from aqueous ammonia, alkanolamines and mixtures thereof, better still from alkanolamines and mixtures thereof, and even better still the alkaline agent is monoethanolamine.

4. Process according to any one of the preceding claims, **characterized in that** the total content of the alkaline agent(s) ranges from 0.01% to 10% by weight, preferably from 0.05% to 8% by weight and more preferentially from 0.1% to 5% by weight, relative to the total weight of the composition comprising them.

5. Process according to any one of the preceding claims, **characterized in that** the oxidation dyes are chosen from oxidation bases, preferably chosen from para-phenylenediamines, bis(phenyl)alkylenediamines, para-aminophenols, ortho-aminophenols, heterocyclic bases, and the corresponding addition salts, the solvates thereof and/or the solvates of the salts thereof, and mixtures thereof; more preferentially para-aminophenol, 2,3-diaminodihydropyrazolopyrazolone, 2-methoxymethyl-para-phenylenediamine, 2-β-hydroxyethyl-para-phenylenediamine, 2-γ-hydroxypropyl-para-phenylenediamine, the addition salts thereof, the solvates thereof, the solvates of the salts thereof, and mixtures thereof.

6. Process according to the preceding claim, **characterized in that** the oxidation dye(s) are also chosen from couplers, preferably chosen from meta-phenylenediamines, meta-aminophenols, meta-diphenols, naphthalene-based couplers and heterocyclic couplers, and also the addition salts thereof, the solvates thereof, the solvates of the salts thereof, and mixtures thereof, more preferentially from 3-amino-6-methoxy-2-methylaminopyridine, 6-hydroxybenzomorpholine, 2,4-diamino-1-(β-hydroxyethyloxy)benzene, 2-amino-3-hydroxypyridine, 5-amino-6-chloro-2-methylphenol, 1-methyl-2-hydroxy-4-β-hydroxyethylaminobenzene, 2-amino-4-hydroxyethylaminoanisole, hydroxyethyl-3,4-methylenedioxyaniline, 2-amino-5-ethylphenol, 1-hydroxy-3-aminobenzene, 4-amino-2-hydroxytoluene, the addition salts thereof, the solvates thereof, the solvates of the salts thereof, and mixtures thereof, more preferentially from 6-hydroxybenzomorpholine, the addition salts thereof, the solvates thereof and/or the solvates of the salts thereof, hydroxyethyl-3,4-methylenedioxyaniline, the addition salts thereof, the solvates thereof and/or the solvates of the salts thereof, 2-amino-5-ethylphenol, the addition salts thereof, the solvates thereof and/or the solvates of the salts thereof, and mixtures thereof.

7. Process according to any one of the preceding claims, **characterized in that** the chemical oxidizing agent(s) are chosen from hydrogen peroxide, urea peroxide, alkali metal bromates, persalts, peracids and oxidase enzymes (with their optional cofactors), and mixtures thereof; more preferentially, the chemical oxidizing agent(s) are chosen from hydrogen peroxide, persalts and mixtures thereof, more preferably hydrogen peroxide.

8. Process according to any one of the preceding claims, **characterized in that** the extemporaneous mixture also comprises an oxidizing composition C comprising one or more chemical oxidizing agents according to Claim 7.

9. Process according to any one of the preceding claims, **characterized in that** the composition B results from the mixing of a composition comprising one or more alkaline agents according to Claim 3 or 4 and one or more dyes chosen from oxidation dyes, direct dyes and mixtures thereof according to Claim 5 or 6 and of an additional oxidizing composition comprising one or more chemical oxidizing agents according to Claim 7.

10. Process according to any one of the preceding claims, **characterized in that** the composition A, the composition B and/or the composition C, preferably the composition A, also comprise one or more cationic polymers preferably chosen from non-associative cationic polymers and mixtures thereof, more preferentially cationic polysaccharides, alkyldiallylamine cyclopolymers or dialkyldiallylammonium cyclopolymers and mixtures thereof, and even better still from guar gums, dimethyldiallylammonium salt homopolymers, copolymers of salts of diallyldimethylammonium and of acrylamide, and mixtures thereof.

11. Process according to any one of the preceding claims, **characterized in that** the composition A, the composition B and/or the composition C also comprise one or more non-ionic surfactants preferably chosen from:
- oxyethylenated C₈ to C₄₀ alcohols comprising from 1 to 100 mol of ethylene oxide, preferably from 2 to 50 and more particularly from 2 to 40 mol of ethylene oxide;
- saturated or unsaturated oxyethylenated plant oils comprising from 1 to 100, preferably from 2 to 70, mol of ethylene oxide;
- and mixtures thereof.

12. Multi-compartment device comprising at least one first compartment containing a composition A according to any one of Claims 1 to 6 and 10 to 11, and at least one second compartment containing a composition B according to any one of Claims 1 and 3 to 6, 10 and 11.

13. Multi-compartment device according to claim 12. **characterized in that** it comprises at least a third compartment containing a composition C according to any one of Claims 8, 10 and 11.

## Patentansprüche

1. Verfahren zum Färben von Keratinfasern, insbesondere menschlichen Keratinfasern, wie z. B. dem Haar, umfassend Aufbringen eines unvorbereiteten Gemischs auf die Keratinfasern:
i) aus wenigstens einer kosmetischen Zusammensetzung A umfassend:
- Propan-1,3-diol, vorhanden in einem Gesamtgehalt von größer als oder gleich 3 Gew.-%, bezogen auf das Gesamtgewicht der Zusammensetzung A,
- ein oder mehrere alkalische Mittel, und
- einen oder mehrere Farbstoffe ausgewählt aus Oxidationsfarbstoffen, Direktfarbstoffen und Gemischen davon; und
ii) aus wenigstens einer färbenden Zusammensetzung B umfassend:
- ein oder mehrere alkalische Mittel, und
- einen oder mehrere Farbstoffe ausgewählt aus Oxidationsfarbstoffen, Direktfarbstoffen und Gemischen davon;
wobei zu beachten ist, dass, wenn die Zusammensetzung A und/oder B wenigstens einen Oxidationsfarbstoff umfasst, das unvorbereitete Gemisch auch ein oder mehrere Oxidationsmittel umfasst; wobei die Oxidationsmittel in der Zusammensetzung B und/oder in einer oxidierenden Zusammensetzung C vorhanden sind.

2. Verfahren nach Anspruch 1, **dadurch gekennzeichnet, dass** der Gesamtgehalt an Propan-1,3-diol in dem Bereich von 3 Gew.-% bis 15 Gew.-%, vorzugsweise von 4 Gew.-% bis 10 Gew.-% und bevorzugter von 4 Gew.-% bis 7 Gew.-%, bezogen auf das Gesamtgewicht der Zusammensetzung A, liegt.

3. Verfahren nach einem der vorstehenden Ansprüche, **dadurch gekennzeichnet, dass** die alkalische(n) Mittel ausgewählt sind aus Alkanolaminen, wässrigem Ammoniak, Alkalimetall- oder Erdalkalimetallcarbonaten oder - bicarbonaten, Alkalimetall- oder Erdalkalimetallsilicaten oder -metasilicaten und Gemischen davon, bevorzugter aus wässrigem Ammoniak, Alkanolaminen und Gemischen davon, noch besser aus Alkanolaminen und Gemischen davon und sogar noch besser das alkalische Mittel Monoethanolamin ist.

4. Verfahren nach einem der vorstehenden Ansprüche, **dadurch gekennzeichnet, dass** der Gesamtgehalt an den alkalische(n) Mittel(n) in dem Bereich von 0,01 Gew.-% bis 10 Gew.-%, vorzugsweise von 0,05 Gew.-% bis 8 Gew.-% und bevorzugter von 0,1 Gew.-% bis 5 Gew.-%, bezogen auf das Gesamtgewicht der sie umfassenden Zusammensetzung, liegt.

5. Verfahren nach einem der vorstehenden Ansprüche, **dadurch gekennzeichnet, dass** die Oxidationsfarbstoffe ausgewählt sind aus Oxidationsbasen, vorzugsweise ausgewählt aus para-Phenylendiaminen, Bis(phenyl)alkylendiaminen, para-Aminophenolen, ortho-Aminophenolen, heterocyclischen Basen und den entsprechenden Additionssalzen, den Solvaten davon und/oder den Solvaten der Salze davon und Gemischen davon; bevorzugter para-Aminophenol, 2,3-Diaminodihydropyrazolopyrazolon, 2-Methoxymethyl-paraphenylendiamin, 2-β-Hydroxyethyl-para-phenylendiamin, 2-γ-Hydroxypropyl-para-phenylendiamin, den Additionssalzen davon, den Solvaten davon, den Solvaten der Salze davon und Gemischen davon.

6. Verfahren nach dem vorstehenden Anspruch, **dadurch gekennzeichnet, dass** die Oxidationsfarbstoffe auch ausgewählt sind aus Kopplern, vorzugsweise ausgewählt aus meta-Phenylendiaminen, meta-Aminophenolen, meta-Diphenolen, Kopplern auf Naphthalinbasis und heterocyclischen Kopplern, und auch den Additionssalzen davon, den Solvaten davon, den Solvaten der Salze davon und Gemischen davon, bevorzugter aus 3-Amino-6-methoxy-2-methylaminopyridin, 6-Hydroxybenzomorpholin, 2,4-Diamino-1-(β-hydroxyethyloxy)benzol, 2-Amino-3-hydroxypyridin, 5-Amino-6-chlor-2-methylphenol, 1-Methyl-2-hydroxy-4-β-hydroxyethylaminobenzol, 2-Amino-4-hydroxyethylaminoanisol, Hydroxyethyl-3,4-methylendioxyanilin, 2-Amino-5-ethylphenol, 1-Hydroxy-3-aminobenzol, 4-Amino-2-hydroxytoluol, den Additionssalzen davon, den Solvaten davon, den Solvaten der Salze davon und Gemischen davon, bevorzugter aus 6-Hydroxybenzomorpholin, den Additionssalzen davon, den Solvaten davon und/oder den Solvaten der Salze davon, Hydroxyethyl-3,4-methylendioxyanilin, den Additionssalzen davon, den Solvaten davon und/oder den Solvaten der Salze davon, 2-Amino-5-ethylphenol, den Additionssalzen davon, den Solvaten davon und/oder den Solvaten der Salze davon, und Gemischen davon.

7. Verfahren nach einem der vorstehenden Ansprüche, **dadurch gekennzeichnet, dass** die chemischen Oxidationsmittel ausgewählt sind aus Wasserstoffperoxid, Harnstoffperoxid, Alkalimetallbromaten, Persalzen, Persäuren und Oxidaseenzymen (mit ihren optionalen Cofaktoren) und Gemischen davon; bevorzugter sind die chemischen Oxidationsmittel ausgewählt aus Wasserstoffperoxid, Persalzen und Gemischen davon, bevorzugter Wasserstoffperoxid.

8. Verfahren nach einem der vorstehenden Ansprüche, **dadurch gekennzeichnet, dass** das unvorbereitete Gemisch auch eine oxidierende Zusammensetzung C umfassend ein oder mehrere chemische Oxidationsmittel nach Anspruch 7 umfasst.

9. Verfahren nach einem der vorstehenden Ansprüche, **dadurch gekennzeichnet, dass** die Zusammensetzung B durch Mischen einer Zusammensetzung umfassend ein oder mehrere alkalische Mittel nach Anspruch 3 oder 4 und eines oder mehrerer Farbstoffe ausgewählt aus Oxidationsfarbstoffen, Direktfarbstoffen und Gemischen davon nach Anspruch 5 oder 6 und einer zusätzlichen oxidierenden Zusammensetzung umfassend ein oder mehrere chemische Oxidationsmittel nach Anspruch 7 entsteht.

10. Verfahren nach einem der vorstehenden Ansprüche, **dadurch gekennzeichnet, dass** die Zusammensetzung A, die Zusammensetzung B und/oder die Zusammensetzung C, vorzugsweise die Zusammensetzung A, auch ein oder mehrere kationische Polymere enthalten, vorzugsweise ausgewählt aus nicht assoziativen kationischen Polymeren und Gemischen davon, bevorzugter kationischen Polysacchariden, Alkyldiallylamincyclopolymeren oder Dialkyldiallylammoniumcyclopolymeren und Gemischen davon, und noch besser aus Guargummis, Dimethyldiallylammoniumsalz-Homopolymeren, Copolymeren von Salzen von Diallyldimethylammonium und Acrylamid, und Gemischen davon.

11. Verfahren nach einem der vorstehenden Ansprüche, **dadurch gekennzeichnet, dass** die Zusammensetzung A, die Zusammensetzung B und/oder die Zusammensetzung C ferner ein oder mehrere nichtionische Tenside umfasst, vorzugsweise ausgewählt aus:
- oxyethylenierten C₈- bis C₄₀-Alkoholen umfassend von 1 bis 100 Mol Ethylenoxid, vorzugsweise von 2 bis 50 und insbesondere von 2 bis 40 Mol Ethylenoxid;
- gesättigten oder ungesättigten oxyethylenierten Pflanzenölen umfassend von 1 bis 100, vorzugsweise von 2 bis 70, Mol Ethylenoxid;
- und Gemischen davon.

12. Mehrkammervorrichtung umfassend wenigstens eine erste Kammer, die eine Zusammensetzung A nach einem der Ansprüche 1 bis 6 und 10 bis 11 enthält, und wenigstens eine zweite Kammer, die eine Zusammensetzung B nach einem der Ansprüche 1 und 3 bis 6, 10 und 11 enthält.

13. Mehrkammervorrichtung nach Anspruch 12, **dadurch gekennzeichnet, dass** sie wenigstens eine dritte Kammer umfasst, die eine Zusammensetzung C nach einem der Ansprüche 8, 10 und 11 enthält.

## Revendications

1. Procédé de coloration des fibres kératiniques, en particulier des fibres kératiniques humaines telles que les cheveux, comprenant l'application sur lesdites fibres kératiniques d'un mélange extemporané :
i) d'au moins une composition cosmétique A comprenant :
- du propane-1,3-diol, présent dans une teneur totale supérieure ou égale à 3% en poids, par rapport au poids total de la composition A,
- un ou plusieurs agents alcalins, et
- un ou plusieurs colorants choisis parmi les colorants d'oxydation, les colorants directs et leurs mélanges ; et
ii) d'au moins une composition colorante B comprenant :
- un ou plusieurs agents alcalins, et
- un ou plusieurs colorants choisis parmi les colorants d'oxydation, les colorants directs et leurs mélanges ;
étant entendu que lorsque les compositions A et/ou B comprennent au moins un colorant d'oxydation, le mélange extemporané comprend en outre un ou plusieurs agents oxydants ; le ou lesdits agents oxydants étants présents dans la composition B et/ou dans une composition oxydante C.

2. Procédé selon la revendication 1, **caractérisé en ce que** la teneur totale en propane-1,3-diol va de 3 à 15% en poids, de préférence de 4 à 10% en poids, plus préférentiellement de 4 à 7% en poids, par rapport au poids total de la composition A.

3. Procédé selon l'une quelconque des revendications précédentes, **caractérisé en ce que** le ou les agents alcalins sont choisis parmi les alcanolamines, l'ammoniaque, les carbonates ou les bicarbonates de métaux alcalins ou alcalino terreux, les silicates ou métasilicates de métaux alcalins ou alcalino terreux et leurs mélanges, plus préférentiellement parmi l'ammoniaque, les alcanolamines et leurs mélanges, mieux parmi les alcanolamines et leurs mélanges, mieux encore l'agent alcalin est la monoéthanolamine.

4. Procédé selon l'une quelconque des revendications précédentes, **caractérisé en ce que** la teneur totale du ou des agents alcalins va de 0,01 à 10% en poids, de préférence de 0,05 à 8% en poids, plus préférentiellement de 0,1 à 5% en poids, par rapport au poids total de la composition les comprenant.

5. Procédé selon l'une quelconque des revendications précédentes, **caractérisé en ce que** les colorants d'oxydation sont choisis parmi les bases d'oxydation, de préférence choisies parmi les para-phénylènediamines, les bis(phényl)alkylènediamines, les para-aminophénols, les ortho-aminophénols, les bases hétérocycliques, et les sels d'addition correspondants, leurs solvates et/ou les solvates de leurs sels et leurs mélanges ; plus préférentiellement la para-aminophénol, la 2,3-diaminodihydropyrazolo pyrazolone, la 2-méthoxyméthyl-para-phénylènediamine, la 2-β-hydroxyéthyl-para-phénylènediamine, la 2-γ-hydroxypropyl-para-phénylènediamine, leurs sels d'addition, leurs solvates, les solvates de leurs sels et leurs mélanges.

6. Procédé selon la revendication précédente, **caractérisé en ce que** le ou les colorants d'oxydation sont en outre choisis parmi les coupleurs, de préférence choisis parmi les métaphénylènediamines, les méta-aminophénols, les méta-diphénols, les coupleurs naphtaléniques, les coupleurs hétérocycliques ainsi que leurs sels d'addition, leurs solvates, les solvates de leurs sels et leurs mélanges, plus préférentiellement parmi la 3-amino-6-methoxy-2-methylamino pyridine, la 6-hydroxy benzomorpholine, le 2,4-diamino-1-(β-hydroxyéthyloxy) benzène, la 2-amino-3-hydroxy pyridine, le 5-amino-6-chloro-2-méthyl phénol, le 1-méthyl-2-hydroxy-4-β-hydroxyéthylamino benzène, la 2-amino 4-hydroxyethylaminoanisole, l'hydroxyéthyl-3,4-méthylènedioxyaniline, le 2-amino 5-éthylphénol, le 1-hydroxy 3- amino benzène, le 4-amino-2-hydroxy toluène, leurs sels d'addition, leurs solvates, les solvates de leurs sels et leurs mélanges, plus préférentiellement parmi le 6-hydroxy benzomorpholine, ses sels d'addition, ses solvates et/ou les solvates de ses sels, l'hydroxyéthyl-3-4-méthylènedioxyaniline, ses sels d'addition, ses solvates et/ou les solvates de ses sels, le 2-amino 5-éthylphénol, ses sels d'addition, ses solvates et/ou les solvates de ses sels et leurs mélange.

7. Procédé selon l'une quelconque des revendications précédentes, **caractérisé en ce que** le ou les agents oxydants chimiques sont choisis parmi le peroxyde d'hydrogène, le peroxyde d'urée, les bromates de métaux alcalins, les persels, les peracides et les enzymes oxydases (avec leurs cofacteurs éventuels), et leurs mélanges ; plus préférentiellement, le ou les agents oxydants chimiques sont choisis parmi le peroxyde d'hydrogène, les persels, et leurs mélanges, de préférence encore le peroxyde d'hydrogène.

8. Procédé selon l'une quelconque des revendications précédentes, **caractérisé en ce que** le mélange extemporané comprend en outre une composition oxydante C comprenant un ou plusieurs agents oxydants chimiques selon la revendication 7.

9. Procédé selon l'une quelconque des revendications précédentes, **caractérisé en ce que** la composition B est issue du mélange d'une composition comprenant un ou plusieurs agents alcalins selon les revendications 3 ou 4 et un ou plusieurs colorants choisis parmi les colorants d'oxydation, les colorants directs et leurs mélanges selon les revendications 5 ou 6 et d'une composition oxydante additionnelle comprenant un ou plusieurs agents oxydants chimiques selon la revendication 7.

10. Procédé selon l'une quelconque des revendications précédentes, **caractérisé en ce que** la composition A, la composition B et/ou la composition C, de préférence la composition A, comprennent en outre un ou plusieurs polymères cationiques choisis de préférence parmi les polymères cationiques non associatifs et leurs mélanges, plus préférentiellement les polysaccharides cationiques, les cyclopolymères d'alkyl diallyl amine ou de dialkyl diallyl ammonium et leurs mélanges, et mieux encore parmi les gommes de guar, les homopolymères de sels de diméthyldiallylammonium, les copolymères de sels de diallyldiméthylammonium et d'acrylamide, et leurs mélanges.

11. Procédé selon l'une quelconque des revendications précédentes, **caractérisé en ce que** la composition A, la composition B et/ou la composition C comprennent en outre un ou plusieurs tensioactifs non ioniques choisis de préférence parmi :
- les alcools en C₈ à C₄₀, oxyéthylénés comprenant de 1 à 100 moles d'oxyde d'éthylène, de préférence de 2 à 50, plus particulièrement de 2 à 40 moles d'oxyde d'éthylène ;
- les huiles végétales oxyéthylénées, saturées ou non comprenant de 1 à 100 moles d'oxyde d'éthylène, de préférence de 2 à 70 ;
- et leurs mélanges.

12. Dispositif à plusieurs compartiments comprenant au moins un premier compartiment renfermant une composition A selon l'une quelconque des revendications 1 à 6 et 10 à 11, et au moins un deuxième compartiment renfermant une composition B selon l'une quelconque des revendications 1 et 3 à 6, 10 et 11.

13. Dispositif à plusieurs compartiments selon la revendication 12 **caractérisé en ce qu'**il comprend au moins un troisième compartiment renfermant une composition C selon l'une quelconque des revendications 8, 10 et 11.
